# EUROPÄISCHE PATENTSCHRIFT

(11) **EP 4 558 208 B1**
(45) Veröffentlichungstag und Bekanntmachung des Hinweises auf die Patenterteilung: **03.06.2026**
(21) Anmeldenummer: 23748035.5
(22) Anmeldetag: 21.07.2023
(51) Int. Cl.: A61M 25/10

(54) **BALLONKATHETERVORRICHTUNQ ZUR ATRAUMATISCHEN AUFDEHNUNG VON HOHLORGANEN SOWIE EIN VERFAHREN ZUR HERSTELLUNG EINER SOLCHEN BALLONKATHETERVORRICHTUNG**
BALLOON CATHETER DEVICE FOR ATRAUMATIC EXPANSION OF HOLLOW ORGANS, AND A METHOD FOR PRODUCING SUCH A BALLOON CATHETER DEVICE
DISPOSITIF DE CATHÉTER À BALLONNET POUR LA DILATATION ATRAUMATIQUE D'ORGANES CREUX ET PROCÉDÉ DE FABRICATION D'UN TEL DISPOSITIF DE CATHÉTER À BALLONNET

(30) Priorität: 22.07.2022 DE 102022118449; 06.09.2022 DE 102022122630
(43) Veröffentlichungstag der Anmeldung: 28.05.2025
(73) Patentinhaber: BVS - Best Vascular Solutions GmbH, 53173 Bonn (DE)
(72) Erfinder: BROHM-SCHMITZ-RODE, Andrea, 52070 Aachen (DE); SCHMITZ-RODE, Thomas, 52070 Aachen (DE)
(74) Vertreter: HGF
(86) Internationale Anmeldenummer: PCT/EP2023/070364
(87) Internationale Veröffentlichungsnummer: WO 2024/018082

(56) Entgegenhaltungen:
- WO-A1-2016/151035
- US-A- 5 810 767
- US-A1- 2006 135 985
- US-A1- 2014 066 960

## Beschreibung

Die vorliegende Erfindung betrifft eine Ballonkathetervorrichtung (Ballonkatheter mit Funktionshülse) sowie ein Verfahren zur Herstellung einer solchen Ballonkathetervorrichtung mit Funktionshülse. Eine Aufdehnung von Engstellen von Hohlorganen mittels Ballonkatheter ist fester Bestandteil einer minimal-invasiven Therapie. Dies betrifft insbesondere die Blutgefäße. Im Rahmen der sog. endovaskulären Therapie werden Atherosklerose-bedingte Engstellungen und Verschlüsse von Blutgefäßen durch Ballonaufdehnung behandelt.

Typischerweise wird dazu ein Ballonkatheter unter Bildgebungskontrolle in das Gefäßsystem eingeführt. Nach Platzierung des Ballons im Bereich der zu behandelnden Läsion wird der Ballon unter hohem Druck aufgedehnt. Diese Standardbehandlung kann jedoch mit gravierenden Komplikationen verbunden sein. Durch die Ballonaufdehnung wird die Gefäßwand verletzt. Typischerweise entstehen Längsrisse in der Gefäßwand. Diese Verletzung kann in den ersten Tagen nach Behandlung eine Gerinnselanlagerung zur Folge haben. In den folgenden Tagen bis zu einem Zeitraum von etwa drei Monaten reagiert die Blutgefäßwand auf das Dilatationstrauma mit einer überschießenden Wandreaktion. Glatte Muskelzellen in der Gefäßwand werden durch das Trauma stimuliert und produzieren extrazelluläre Matrix ("intimale Hyperplasie"). Die damit verbundene Volumenzunahme in der Gefäßwand führt zu einer erneuten Ausbildung einer Verengung und wirkt damit dem Behandlungsergebnis entgegen. Auch die Implantation einer Gefäßstütze (Stent) zur dauerhaften Gefäßaufweitung kann dies nicht verhindern. Ein Stent ist ein Fremdkörper, der einerseits gerinnselbildend wirken kann, andererseits durch seine Steifigkeit die natürliche pulsatile Motilität des Stent-versorgten Gefäßabschnitts verhindert und so einen ständigen Reiz auf die Gefäßwand ausübt. Dieser Reiz kann wiederum zur überschießenden Produktion der Gefäßwandzellen mit Einwachsen des Gewebes durch die Stentstreben und damit zur Ausbildung einer erneuten Verengung führen.

Daher sind die heute üblicherweise implantierten Stents mit einem die Zellstimulation und Matrixproduktion reduzierenden ("antiproliferativen") Wirkstoff beschichtet. Im Falle einer alleinigen Ballondilatation ohne Stentimplantation werden heute überwiegend Ballonkatheter eingesetzt, deren Ballonhülle außen mit einem vergleichbaren antiproliferativen Wirkstoff beschichtet ist, der bei Ballonaufdehnung in die Gefäßwand übertragen werden soll. Üblicherweise besteht die Beschichtung aus einem homogenen filmartigen Überzug der äußeren Ballonmembran, der den Wirkstoff eingebettet in ein Trägermaterial (Bindemittel, "Exipient") enthält. Das therapeutische Ziel ist die Übermittlung einer therapeutischen Wirkstoffdosis in die Gefäßwand für den kritischen Zeitraum von 6 Wochen bis max. 3 Monaten zur Unterdrückung der überschießenden Gefäßwandreaktion (Katsanos et al., J Am Heart Assoc 2018).

Insbesondere in der WO 2016/151035 A1 ist eine rohrförmige Hülse zur atraumatischen Behandlung von Hohlorganen beschrieben. Dabei ist vorgesehen die Hülse mittels eines Ballonkatheters abzuwerfen, sodass die Hülse als Implantat im Hohlorgan verbleibt. Somit erfolgt während der Ballonentfaltung eine Trennung der Hülse von der Ballonmembran.

In der US 2014/0239558 A1 ist eine Kombination aus "injection molding" und "blow molding" mit anschließend Laserschneiden beschrieben. Auf diese Weise soll ein Polymer-Stent wie der sogenannte "Esprit Stent" ausgebildet werden, der wie ein normaler Stent seine Zellen öffnet, wenn er auf einem Ballon entfaltet wird. Weiterhin ist ein Abwerfen des Stents von einem Ballon nach einer Ballonkatheterentfaltung vorgesehen.

Aus der US 2005/0182361 A1 geht eine Hülse aus einem elastischen Material hervor zum Schutz einer darunter liegenden Wirkstoffschicht. Dabei ist die elastische Hülse über den gefalteten Ballon des Ballonkatheters gestülpt. Die ungefaltete Hülse liegt elastisch über der gefalteten Ballonmembran und wird durch den sich entfaltenden Ballon gedehnt. Bereits im Zuge der Ballonentfaltung kommt es zu einer wesentlichen Umfangsvergrößerung der Hülse, wobei sich aus Schlitzen rautenartige Fenster bilden. Zweck der Vorrichtung ist ein Schutz der Wirkstoffschicht.

In der CN 1 11 107 889 A ist ein Ballonkatheter offenbart auf dem eine Abdeckung mit einer medizinischen Wirkstoffschicht angeordnet ist. Die Wirkstoffschicht bzw. die Abdeckung ist von einer Hülse umgeben, um den Wirkstoff zu schützen. Die Hülse kann mit einem Katheterschaft verbunden sein. Die Abdeckung und die Hülse sind nicht gefaltet, lediglich der Ballon ist gefaltet. Hierbei ist die Abdeckung rollbar ausgebildet, um eine Applikation von unterschiedlichen Wirkstoffen zu ermöglichen.

Aus der EP 3 922 217 A1 geht eine rohrförmige Vliesstruktur als Wirkstoffträger (kurz "Hülse" genannt) zur atraumatischen Behandlung von Hohlorganen, insbesondere für eine Ballondilatation, hervor, wobei die Hülse in einem Ausgangszustand um eine Hülsenlängsachse gefaltet ist und in einem Endzustand zum Anliegen an einer Innenwandung eines Hohlorganes entfaltbar ist, wobei die rohrförmige Hülse aus zumindest ersten biodegradierbaren Polymer-Nano-Fasern ausgebildet ist und die Faltung der Hülse als Plissierung um eine Hülsenlängsachse gerichtet ist, wobei ein medizinischer Wirkstoff in die ersten Polymer-Nano-Fasern eingelagert und /oder in Zwischenräumen zwischen den Polymer-Nano-Fasern angeordnet ist, und wobei die ersten Polymerfasern derart ausgebildet sind, dass sie als langsam biodegradierbare Polymer-Nanofasern (PL) über einen einstellbaren Zeitraum von 2 Wochen bis zu 3 Monaten degradieren, sodass der Wirkstoff in diesem Zeitraum an eine Hohlorganwandung abgebbar ist. Die rohrförmige Hülse ist aus zumindest den ersten und zweiten biodegradierbaren Polymer-Nano-Fasem ausgebildet.

In der US 11,000,680 B2 ist eine Ballonkathetervorrichtung mit einem dehnbaren Ballon, der von einem "Käfig" aus axial und tangential verlaufenden Nitinol-Drähten umgeben ist, beschrieben. Dieser Käfig erzeugt bei Aufdehnung der Ballonmembran multiple kleine Kissen, die eine besser dosierte Kraftverteilung auf die Gefäßwand ausüben sollen und damit gegenüber einem herkömmlichen Ballon das Gefäßwand-Trauma reduzieren soll.

In der US 5 810 767 A ist ein Verfahren offenbart, wobei ein intravaskulärer Katheter Flüssigkeitsinfusionsschläuchen mit einer Ballonoberfläche umfasst und bei Aufblasen des Ballons eine Vielzahl von isolierten Reservoirtaschen definiert. Daraus resultiert, dass sich der Flüssigkeitsverlust in einem einzigen Seitenarm in der Regel auf eine oder höchstens zwei der isolierten Vorratstaschen beschränkt und der Druck in den übrigen Vorratstaschen ohne wesentliche Beeinträchtigung aufrechterhalten werden kann.

Die US 2014/066960 A1 schreibt ein System, umfassend ein Ballon, welcher auf einem Katheters angebracht ist, und eine ausziehbare Struktur, welche über die Ballon angebracht ist und eine Vielzahl von axialen und radialen Stabilisationselementen. Dadurch kann der Ballon so eingespannt werden, dass isolierte Ballonbereiche durch Öffnungen der aus dehnbaren Struktur herausragen, wenn der Ballon aufgeblasen wird.

Aus der US 2006/135985 A1 geht ein Verfahren und eine Vorrichtung zum Einführen eines ausdehnbaren Körpers in ein Blutgefäß, das Ausdehnen des ausdehnbaren Körpers von einem ersten Durchmesser auf einen davon unterschiedlichen zweiten Durchmesser, und die Form des Innendurchmessers des Blutgefäßes an einer Läsion zu verändern, ohne dass die Läsion reist.

Ähnliche Vorrichtungen und Verfahren sind aus der DE 10 2012 007 640, der WO 02 076 700 A1, der US 5 443 495 A1, der DE 10 2006 020 687 A1, der US 2005 / 0 090 888 A1, der DE 2005 056 529, der US 2002 / 0 045 930 A1, der US 2005 /0 125 053 A1, der US 2008 / 0 262 594, der US 5,507,770 A, der US 6,059,823 A und der US 2010/249946 A1 bekannt.

Aufgabe der vorliegenden Erfindung ist es, ein medizinisches Instrument bzw. eine Vorrichtung zur atraumatischen Behandlung von Hohlorganen bereitzustellen, welches eine Alternative zu den aus dem Stand der Technik bekannten medizinischen Vorrichtungen darstellt.

Aus der WO 2016/151035 A1 und der EP 3 922 217 A1 geht eine rohrförmige Hülse und eine rohrförmige Vliesstruktur als Wirkstoffträger hervor, die mittels eines Ballons eines Ballonkatheters abwerfbar ist.

Zudem soll ein Verfahren zur Herstellung eines solchen medizinischen Instruments bereitgestellt werden.

Diese Aufgaben werden mit einer Vorrichtung gemäß dem Patentanspruch 1 und Verfahren gemäß den Patentansprüchen 14 und 15 gelöst. Vorteilhafte Ausgestaltungen davon sind in den Unteransprüchen angegeben.

Erfindungsgemäß ist eine Ballonkathetervorrichtung zur atraumatischen Aufdehnung von Hohlorganen vorgesehen. Diese umfasst
einen Ballonkatheter mit einem Ballon,
eine auf dem Ballon applizierte rohrförmige ebenflächige Funktionshülse zur gerichteten segmentierten kissenartigen Entfaltung des Ballons.

Die Erfindung zeichnet sich dadurch aus, dass die Funktionshülse in einem Ausgangszustand zusammen mit dem Ballon um eine Hülsenlängsachse gefaltet ist, und wobei die Faltung der Funktionshülse als Plissierung um eine Hülsenlängsachse gerichtet ist und in einem Endzustand zum Anliegen an einer Innenwandung eines Hohlorgans entfaltbar ist, und wobei die rohrförmige Funktionshülse ebenflächige Abschnitte und/oder ebenflächige Streben aufweist, die Durchgangsöffnungen begrenzen, so dass die Funktionshülse zur abschnittsweisen Begrenzung einer Ballonaufdehnung des Ballons, und wobei die Funktionshülse im Bereich der ebenflächigen Abschnitte und/oder der ebenflächigen Streben mit dem Ballon verbunden ist.

Unter einer atraumatischen Behandlung von Hohlorganen kann zum einen eine Aufdehnung eines Hohlorgans verstanden werden, bei der ein Hohlorgan aufgedehnt wird, Zum anderen kann eine zeitlich begrenzte Implantation vorgesehen sein, bei der ein medizinischer Wirkstoff appliziert wird. Eine solche zeitlich begrenzte Applikation erstreckt sich über einen Zeitraum von zumindest zwei bzw. zumindest drei bis maximal 5 bzw. 10 Minuten über. Eine gleichzeitig damit erfolgende Wirkstoffabgabe in die Hohlorganwand hält i.d.R. über Wochen bis Monate.

Durch die ebenflächigen Abschnitte und/oder ebenflächigen Streben, die die Durchgangsöffnungen begrenzen, ermöglicht die Funktionshülse eine abschnittsweise begrenzte und insbesondere verstärkte Ballonaufdehnung des Ballons, so dass es zu einer gleichmäßigen Kissenbildung des Ballons im Bereich der Durchgangsöffnungen kommt. Auf diese Weise werden (im Vergleich zu herkömmlichen Ballons) ein gleichmäßigeres Anliegen und eine gleichmäßigere Kraftverteilung an bzw. auf die Hohlorgan-bzw. Blutgefäßwand bei der Ballonaufdehnung erzielt. Hierdurch können umschriebene große Traumata der Wandung sowie entsprechende Rissbildungen reduziert werden.

Insbesondere wird durch die ebenflächige Funktionshülse mit Durchgangsöffnungen eine Bildung von lokalen Spannungsspitzen zwischen Ballonhülle und Gefäßwand beim Entfaltungsvorgang reduziert bzw. vermieden, sodass bei der Ballonaufdehnung keine großen Risse mehr in der Gefäßwand entstehen können.

Der Bereich, in dem die Kissen ausgebildet werden, wird im Folgenden als erster Funktionsbereich bezeichnet.

Im Gegensatz zur erfindungsgemäßen Ballonkathetervorrichtung übt ein Drahtkäfig, wie er bspw. in der US 11,000,680 B2 beschrieben ist, aufgrund lokaler Impressionen kombiniert mit Scherungen beim Entfaltungsvorgang ebenfalls ein Trauma auf die Gefäßwand aus. Zudem erhöht der Drahtkäfig signifikant einen Einführungsdurchmesser einer Ballonkathetervorrichtung.

Die erfindungsgemäße Hülse ist mit der Ballonmembran verbunden und ist zusammen mit der Ballonmembran des Ballonkatheters faltbar und entfaltbar. Die erfindungsgemäße Hülse weist jedoch keine Mikrokompartimente, sondern Durchgangsöffnungen auf, die fensterartig und/oder in Form von Schlitzen ausgebildet sind.

Der Vorteil ebenflächiger Streben der Funktionshülse gegenüber einem Drahtgebilde zur Segmentierung der Ballonentfaltung liegt in einer geringeren Profilbildung bei der Einfaltung, so dass ein geringerer Durchmesser eines Einführkatheters vorgesehen werden kann. Ein niedrigprofilierter Einführkatheter erleichtert die Anwendung. Außerdem kann ein Drahtgebilde bei Ballonentfaltung zu zusätzlichen Hohlorgan-Wandschäden durch eine Kombination aus Drahtimpression und Scherung führen.

Die erfindungsgemäße Funktionshülse ist somit derart segmentiert ausgebildet bzw. weist eine gruppierte Anordnung von Durchgangsöffnungen (Fenster, Schlitze) auf, dass die Funktionshülse im entfalteten Zustand auf ihrer Außenkontur gegenüber der Hohlorganwand eine Kontaktfläche in Form von plattenförmigen bzw. ebenflächigen Abschnitten oder Streben aufweist.

Unter dem Ausdruck "ebenflächig" wird im Rahmen der vorliegenden Erfindung verstanden, dass die rohrförmige Hülse selbst und auch die die rohrförmigen Hülsen ausbildenden Streben und Abschnitte eine größere Erstreckung in einer Fläche parallel zur Ballonoberfläche des Ballons im Vergleich zu einer Dicke orthogonal zur Ballonoberfläche aufweisen.

Erfindungsgemäß ist somit vorgesehen, dass die Hülse im Wesentlichen über eine gesamte Länge der Hülse in Axialrichtung mit der Ballonmembran verbunden ist und zusammen mit dieser zunächst gefaltet und anschließend auch zusammen mit der Ballonmembran entfaltet wird. Das bedeutet, die Hülse verbleibt immer auf der Ballonmembran und wird zusammen mit dieser nach einer kissenförmigen Ballonaufdehnung des Hohlorgans, kombiniert mit einer Wirkstoffapplikation in die Hohlorganwand, wieder aus einem Hohlorgan entfernt.

Eine Verbindung zwischen Ballon und Hülse erfolgt vorzugsweise über durch Adhärenz einer wirkstoffhaltigen adhäsiven Schicht, z.B. ein Gel, die sich zwischen dem Ballon bzw. der Ballonmembran und der Funktionshülse angeordnet ist. Zusätzlich und oder alternativ kann eine punktuelle und/oder linienförmige Verbindung durch Laserschweißen, oder eine flächige Verbindung durch Kleben, insbesondere eine Verbindung über alle ebenmäßigen Flächen oder Streben vorgesehen sein. ) -

Die Funktionshülse weist insbesondere eine höhere Steifigkeit bei in etwa gleicher Wanddicke bzw. ein höheres Elastizitätsmodul auf als die Ballonmembran.

Auf diese Weise wird die kissenartige Entfaltung erheblich begünstigt. Das bedeutet, dass die Hülse dehnungssteifer sein soll als die Ballonmembran, um im Bereich ihrer Flächen/Streben einen umfangsbegrenzenden Effekt auf die sich ausdehnende Ballonmembran ausüben zu können. Damit bildet sich der Kisseneffekt ideal aus. Auch eine in etwa gleiche Dehnungssteifigkeit von Hülse und Ballonmembran oder sogar geringfügig weniger ist denkbar.

Insbesondere kann eine medizinische Wirkstoffschicht zwischen dem Ballon des Ballonkatheters und der Funktionshülse angeordnet sein.

Eine entsprechende Abgabe des medizinischen Wirkstoffs an eine Gefäßwandung kann dann im Bereich der Durchgangsöffnungen erfolgen.

Dieser Bereich wird als zweiter Funktionsbereich bezeichnet und entspricht der Größe des ersten Funktionsbereichs.

Die ebenflächigen Abschnitte und/oder ebenflächigen Streben sorgen dafür, dass die medizinische Wirkstoffschicht, die unter der Funktionshülse angeordnet sein kann, insbesondere beim Einfalten der Ballonkathetervorrichtung für eine niedrigprofilige Lagerung und beim Transport zum Wirkort geschützt ist.

Die Hülse weist vorzugsweise einen Durchmesser auf, der in etwa einem Durchmesser des aufgefalteten bzw. expandierten Ballons entspricht.

Die Durchgangsöffnungen können als insbesondere lasergeschnittene Schlitze zur Medikamenten- bzw. Wirkstoffapplikation ausgebildet sein, wobei in der Funktionshülse in einer Axialrichtung der Funktionshülse alternierend Abschnitte mit Axialschlitzen, die als Axialabschnitte bezeichnet werden, und Abschnitte mit Tangentialschlitzen, die als Tangentialabschnitte bezeichnet werden, ausgebildet sein können, und wobei die Axialabschnitte und die Tangentialabschnitte den ersten Funktionsbereich ausbilden und beabstandet zueinander ausgebildet sind.

Durch das Vorsehen von alternierend angeordneten Axialabschnitten mit entsprechenden Axialschlitzen und Tangential- bzw. Radialabschnitten mit entsprechenden Tangentialabschnitten kommt es erfindungsgemäß zu einer konstanten bzw. im Wesentlichen gleichförmigen Aufweitung eines Durchmessers der Hülse und des Ballons in Axialrichtung.

Die Tangentialabschnitte bilden dabei Entkopplungsabschnitte zwischen den Axialabschnitten aus, um einen in etwa konstanten Durchmesser in radialer Richtung über eine im Wesentlichen gesamte Länge der Vorrichtung in Axialrichtung zu ermöglichen.

Insbesondere sind die Axialabschnitte und die Tangentialabschnitte in einer Axialrichtung derart ausgebildet und beabstandet zueinander angeordnet, dass sie einander nicht überlappen.

Weiterhin können die Axialschlitze der Axialabschnitte und die Tangentialschlitze der Tangentialabschnitte derart ausgebildet und angeordnet sein, dass sie einander in Tangentialrichtung nicht überlappen. Das bedeutet weder sind die Axialschlitze versetzt zueinander angeordnet noch sind die Tangentialschlitze versetzt zueinander angeordnet.

Im Stand der Technik sind Schlitze häufig versetzt zueinander angeordnet. Erfindungsgemäß können die Axialschlitze hinsichtlich Länge und Breite gleichartig ausgebildet und tangential umlaufend in etwa gleich beabstandet und tangential fluchtend zueinander angeordnet sein. Weiterhin können erfindungsgemäß die Tangentialschlitze hinsichtlich Länge und Breite gleichartig ausgebildet und tangential umlaufend in etwa gleich beabstandet und tangential fluchtend zueinander angeordnet sein.

Die Erfinder der vorliegenden Erfindung haben erkannt, dass eine solche, entlang einer Hülsenachse bzw. in Axialrichtung alternierende Anordnung von gleichartigen Axial- und Tangentialschlitzen eine optimale bzw. verbesserte Kissenbildung ermöglicht. Da die Ballonkathetervorrichtung eine zylindrische Form aufweist, ist die Wandspannung in der Funktionshülse unter Ballondruck in Tangentialrichtung doppelt so hoch ist wie in Axialrichtung (Kesselformel). Die Axialschlitze weiten sich unter Balloninnendruck auf. Die eher geringe Aufweitung jedes einzelnen Axialschlitzes summiert sich auf dem Hülsenumfang, sodass insgesamt eine wirksame kissenartige Durchmesseraufweitung resultiert. Im Bereich der Tangentialschlitze erfolgt keine wesentliche Aufweitung, somit resultiert dort auf dem Hülsenumfang eine relative Einschnürung. Eine Wirkstoffapplikation kann aber dennoch über die Tangentialschlitze erfolgen, wie natürlich auch im Bereich der Axialschlitze.

Durch die durch die Tangentialabschnitte ausgebildeten Entkopplungsabschnitte erfolgt eine Einschnürung des Ballons in diesen Bereichen, sodass ein im Wesentlichen konstanter Durchmesser in den Axialabschnitten bereitgestellt wird. Dies führt zu einer gleichmäßigen Verteilung der Kräfte in radialer Richtung nach außen. Auf diese Weise wird ein daraus resultierendes Trauma des Hohlorgans so gering wie möglich gehalten, da es lediglich zu kleineren und gleichmäßiger verteilten Rissen in der Wandung des Hohlorgans kommt.

Im Gegensatz zu bekannten Vorrichtung, bei denen es zu einer bauchigen oder ballonartigen bzw. in etwa kugelförmigen Aufdehnung kommt, kommt es bei der erfindungsgemäßen Vorrichtung zu einer in Axialrichtung im Wesentlichen zylindrischen Aufweitung.

Hierbei kann vorgesehen sein, dass die Hülse in Axialrichtung alternierend Axialabschnitte und Tangentialabschnitte aufweist.

Dadurch, dass der Ballon und die Hülse in ihren Verbindungsbereichen miteinander verbunden sind, ist die Ballonkathetervorrichtung derart ausgebildet, dass sich die Funktionshülse und der Ballon bei einer Ballondeflation und beim Entfernen der Hülse aus einem Hohlorgan, gemeinsam durch das Falten entlang ausgebildeter Faltungslinien zusammengefaltet werden bzw. zusammenfaltbar sind.

Die gesamte Funktionshülse und somit die die Funktionshülse ausbildenden ebenflächigen Streben bzw. ebenflächigen Abschnitte können Poren aufweisen, die die Aufgabe haben bzw. ausgebildet sind, um den darunter befindlichen Wirkstoff bei Entfaltung nach außen an die Hohlorganwand abzugeben, jedoch keinen kissenbildenden Effekt haben.

Die Poren können einen Durchmesser von in etwa 50µm bis 100µm aufweisen.

Eine entsprechende Abgabe des medizinischen Wirkstoffs an eine Gefäßwandung kann dann im gesamten Bereich der Mantelfläche, also im sowohl im Bereich der Durchgangsöffnungen bzw. im Bereich der Kissen als auch über die Poren im Bereich der ebenflächigen Streben erfolgen.

Der zweite Funktionsbereich erstreckt sich gemäß einer solchen Ausführungsform über die gesamte Mantelwandung der Funktionshülse und ist daher in vorteilhafter Weise entsprechend vergrößert, sodass der medizinische Wirkstoff über eine größere Fläche und/oder sodass mehr Wirkstoff abgegeben werden kann.

Die medizinische Wirkstoffschicht kann zusammen mit einem Polymer, beispielsweise einem biodegradierbaren Polymer, oder in Form einer Vliesstruktur aus einem Polymer, bspw. einem biodegradierbaren Polymer ausgebildet sein.

Dann wird die Integrität dieser Schicht bzw. einer Vliesstruktur beim Faltungs- und Entfaltungsvorgang durch die Funktionshülse geschützt.

Vorzugsweise kann der medizinische Wirkstoff in einer Polymer-Gel-Formulierung enthalten sein, die die medizinische Wirkstoffschicht ausbildet.

Dies ist dahingehend vorteilhaft, dass die Polymer-Gel-Formulierung bei Blutserumkontakt geliert und an Volumen zunimmt, sodass der medizinische Wirkstoff bei Ballonentfaltung zwischen Ballonmembran und Funktionshülse über deren Durchgangsöffnungen ausgepresst und so effizient in die Hohlorganwand übertragen werden kann.

Hierbei wird die Integrität dieser Gelschicht beim Faltungsvorgang, der Lagerung und dem Transport zum Wirkort ebenfalls durch die Funktionshülse geschützt.

Dabei ist vorgesehen, dass der Ballon und die Funktionshülse gemeinsam gefaltet werden und im Bereich eines Blutgefäßes entfaltet werden. Nach einer Gefäßaufdehnung durch kissenartige Ballonaufdehnung und einer Wirkstoffübertragung in die Gefäßwand im Bereich der Durchgangsöffnungen und ebenso über die Poren und somit über die gesamte Mantelwandung der Funktionshülse, werden die Funktionshülse und der Ballon gemeinsam wieder gefaltet und entfernt.

Vorzugsweise kann die Funktionshülse hierbei aus einem dehnfesten Polymerfilm, bspw. aus UHWPE Polyethylen oder aus non-compliant Pebax^{®} ausgebildet sein.

Der Ballon selbst kann bspw. aus semi-compliant bzw. compliant Pebax^{®} ausgebildet sein.

Die Durchgangsöffnungen können als Schlitze bzw. langgestreckte Öffnungen, insbesondere Axialschlitze oder sinusförmige axial orientierte Schlitze und/oder als in etwa rechteckförmige oder in etwa elliptische oder in etwa kreisförmige Fenster bzw. fensterartige Öffnungen in einer ansonsten (bis auf die Poren, sofern vorhanden) durchgehend ebenflächigen Mantelwandung der Funktionshülse ausgebildet sein.

Vorzugsweise weisen die Durchgangsöffnungen abgerundete Ecken auf, um eine Rissbildung durch Kerbwirkung zu verhindern.

Zudem können entlang der Hülsenlängsachse zwei oder drei oder vier oder fünf oder mehr Reihen von (vorzugsweise axial orientierten) Schlitzen angeordnet sein.

Zusätzlich und/oder alternativ kann die Hülse über Abschnitte bzw. abschnittsweise oder über ihre gesamte Fläche mit Fenstern versehen sein.

Zwischen zwei oder mehreren sich in Axialrichtung erstreckenden, benachbarten Schlitzen können Sollreißstellen ausgebildet sein, um bei der Lagerung und dem Transport der Ballonkathetervorrichtung die Vorteile einer möglichst geschlossenen Funktionshülse mit den Vorteilen einer größeren Kissenbildung bei der Anwendung zu kombinieren.

Durch das Vorsehen von axialen Sollreißstellen im Bereich zwischen zwei oder mehr benachbarten Axialschlitzen ist somit eine größere Kissenbildung möglich, auch wenn lediglich kurze Axialschlitze vorgesehen sind.

Axial orientierte schlitzartige Durchgangsöffnungen verstärken die Kissenbildung (d.h. Vorwölbung der Ballonmembran nach außen) wohingegen tangential orientierte schlitzartige Durchgangsöffnungen praktisch nicht zu einer Kissenbildung beitragen (relative Einschnürung der Ballonmembran). Die physikalische Erklärung liefert die sogenannte Kesselformel, nach der die tangentiale Wandspannung, die bei Ballonaufdehnung senkrecht zu den Axialschlitzen der Funktionshülse einwirkt, doppelt so groß ist, wie die axiale Wandspannung. Axial verlaufende Schlitze werden unter Innendruck geweitet, gruppiert auf dem Umfang summiert sich dieser Effekt. Tangential verlaufende Schlitze werden hingegen kaum geweitet.

Im Bereich der Durchgangsöffnung bildet die Funktionshülse einen entsprechenden ersten Funktionsbereich zur Kissenbildung aus. Die Wirkstoffabgabe erfolgt über den zweiten Funktionsbereich der entweder dem ersten entspricht oder vorzugsweise die gesamte Mantelwandung der Funktionshülse inklusive Durchgangsöffnungen umfasst, sofern die Funktionshülse Poren aufweist.

Zwischen der Funktionshülse und dem Ballon kann die medizinische Wirkstoffschicht mit einem medizinischen Wirkstoff angeordnet sein, wobei die medizinische Wirkstoffschicht als
eine Beschichtung der Ballonmembran (z.B. durch Dip-Coating) aus einem, insbesondere biodegradierbaren Polymer (oder einer Kombination von Polymeren) und einem medizinischen Wirkstoff, und/oder einem auf der Ballonmembran aufgesprühten, insbesondere biodegradierbaren, Polymer-Vlies mit dem medizinischen Wirkstoff, und/oder
als rohrförmige Zwischenhülse mit einer Vliesstruktur aus, insbesondere biodegradierbarem, Polymer und dem medizinischen Wirkstoff ausgebildet ist, wobei der medizinische Wirkstoff dann in Mikrokompartimente zwischen den Polymerfasern eingelagert ist, und wobei dies vorteilhaft in Verbindung mit entsprechenden fensterartigen Durchgangsöffnungen ist, und/oder
wobei der medizinische Wirkstoff in einer Polymer-Gel-Formulierung als Wirkstoffschicht enthalten ist.

Der medizinische Wirkstoff kann somit in einer Polymer-Gel-Formulierung enthalten sein, die nach Beschichtung der Ballonmembran mit dem Verdunsten des Lösungsmittels trocknet, mit dem Ballon trocken gelagert wird und bei Applikation durch Wasser- bzw. Blutserumkontakt geliert und an Volumen zunimmt, wobei reine Hydrogele oder auch Kombinationen mit Oleo-Gelen (Organo-Gelen) verwendet werden können.

Die über den medizinischen Wirkstoff angeordnete Funktionshülse dient somit auch als Schutz der Wirkstoffschicht bei Einfaltung des Ballons zur Lagerung und dem späteren Transport zum Wirkort, so dass bei Flüssigkeitskontakt und Ballonaufdehnung das wirkstoffhaltige, insbesondere biodegradierbare, Polymer im Bereich der Durchgangsöffnungen der Hülse (Fenster, Schlitze und Poren) nach außen abgepresst und in eine Gefäßwand applizierbar ist.

Dieser Wirkstofftransport durch Abpressen durch die sich entfaltende Ballonmembran durch die Durchgangsöffnungen der Funktionshülse auf ihre Außenfläche ist besonders wirksam, wenn der medizinische Wirkstoff in einer Polymer-Gel-Formulierung enthalten ist, die bei Blutserumkontakt geliert und an Volumen zunimmt. Nach Volumenzunahme der Gelschicht bewirkt die Kompression durch die Ballonmenbran ein Abpressen des Gels durch die Durchgangsöffnungen der Hülse in die Hohlorganwand.

Sofern die medizinische Wirkstoffschicht mit biodegradierbaren Polymer-Nano-Fasern ausgebildet ist, lösen sich diese bei Ballonaufdehnung im Bereich der Durchgangsöffnungen vom Ballon und bilden (Micro-)Flakes aus, die dann an die Hohlorganwand (speziell Gefäßwand) gepresst werden.

Nach der Ballondeflatierung haften Gelschicht oder Fasern an der Hohlorganwand, wobei der Wirkstoff im Gel oder in den degradierbaren Fasern eingeschlossen ist und im Verlauf des biologischen Abbaus (Degradation) des Gels oder der Fasern den therapeutischen wirksamen Wirkstoffspiegel für einen kritischen Zeitraum im Kontakt zu der Hohlorganwand liefert und gleichzeitig auch die nach Ballondilatation entstandenen Längsrisse in der Hohlorganwand teilweise abdichtet. Auf diese Weise lässt sich die Wirkstoffabgabe und auch deren Konzentration über die Zeit optimal auf den Anwendungsfall einstellen.

Durch eine Wirkstoffabgabe über den Abbau des biodegradierbaren Gels oder der Polymer-Nano-Fasern wird die therapeutisch erforderliche Zeitphase mit einem therapeutisch wirksamen Dosierspiegel abgedeckt. Die Biodegradation des Gels bzw. der Abbau der Fasern und somit die Wirkstoffabgabe soll abgeschlossen sein, wenn die kritische Phase vorbei ist, so dass keine permanenten Rückstände im Gefäß verbleiben.

Unter dem Ausdruck "biodegradierbar" (bio-abbaubar) wird im Rahmen der vorliegenden Erfindung verstanden, dass ein Kontakt des Polymer-Gels oder der Polymer-Nano-Fasern mit Körperflüssigkeit und einer Hohlorganwandung (speziell: Blut- und Gefäßwand) einen Zersetzungs- bzw. Abbauprozess dieses Polymers induziert.

Vorzugsweise kann die Faltung der rohrförmigen Funktionshülse als Plissierung ausgebildet sein bzw. die Hülse plissiert sein. Unter "plissieren" wird im Rahmen der vorliegenden Erfindung eine Faltung der Hülse in 2 bis 5 (vorzugsweise 3) gleich große auf den Umfang gleichmäßig verteilte (vorzugsweise 3 x 120°), in Axialrichtung verlaufende Falten verstanden, wobei die Falten gleichmäßig und gleichsinnig um die Längsachse des Ballonkatheters gewunden und an diesen angelegt werden. Durch das Plissieren ist es möglich, den Außendurchmesser der rohrförmigen Hülse im nicht-entfalteten Zustand zu reduzieren.

Als biodegradierbares Polymer können erste und/oder zweite Polymer-Nano-Fasern als biodegradierbares Polymer vorgesehen sein.

Die ersten Polymer-Nano-Fasern können aus langsam degradierbaren Polymer-Fasern (PL) ausgebildet sein. Die zweiten Polymer-Nano-Fasern können aus schnell degradierbaren Polymer-Fasern (PS) ausgebildet.

Vorzugsweise kann nur in das langsam degradierbare Polymer (PL) ein Wirkstoff eingelagert sein. In das schnell degradierende Polymer (PS) kann eventuell zusätzlich ein gerinnungshemmender Wirkstoff wie z.B. Heparin oder ein Antikoagulanz eingelagert sein.

Das degradierbare Polymer kann vorzugsweise hydrophile und/oder lipophilere Eigenschaften aufweisen. Die Degradationsgeschwindigkeit hängt ab von der Polymerart (z.B. bei PLGA-Poly-Lactid-Co-Glycolid) durch das Verhältnis von GA zu LA (ein höherer Gehalt an LA bewirkt eine Reduktion der Hydrophilität und führt daher zu einer langsameren Auflösung), der Molekülkettenlänge (ein hohes Molekulargewicht bzw. eine längere Kettenlänge bewirkt eine langsamere Auflösung), und der Hydrophilität der Seitengruppen (z.B. wirken die Methyl-Seitengruppen in PLA hydrophob und verzögem damit die Auflösung, oder bei PLGA führt eine hydrophil wirkende Carboxylgruppe zu einer schnelleren Auflösung als eine Estergruppe).

Somit ist vorgesehen, dass die medizinische Wirkstoffschicht im Bereich der Durchgangsöffnungen durch die biodegradierbaren Polymer-Gele in multiple Spots oder anhand von biodegradierbaren Polymer-Nano-Fasern ausgebildeten Teilen in multiple "Flakes" bzw. "Microflakes" zerfällt, die flach bzw. ebenflächig sind und an der Hohlorganwand anhaften.

Das biokompatible Polymer der Polymer-Gele oder der Polymer-Nano-Fasern kann aus Polymeren auf Basis von Milchsäure (Polylactid, PLA), Glycolsäure (Polyglycolid, PGA) und ihrer Copolymere (Poly(lactid-co-glycolid), - PLGA), sowie Poly(ε-caprolacton), Polyethylenglycol, Polyethylenoxid, Polysebacinsäure, Po-ly(trimethylencarbonat), Poly(ethylen-co-vinylacetat), Poly(1,5-dioxepan-2-on), Polyvinylpyrrolidon (PVP), Poly-p-dioxanone (PPDX) sowie deren Verbindungen und Copolymeren oder Mischungen daraus ausgebildet sein.

Polymer-Nano-Fasern weisen vorzugsweise einen Faserdurchmesser kleiner ein Mikrometer und vorzugsweise im Bereich von 300 bis 2000 nm und insbesondere im Bereich von 500 bis 1000 nm auf. Die Polymer-Nano-Fasern können im Rahmen der vorliegenden Erfindung auch einen Durchmesser bis zu 3 Mikrometer aufweisen.

Diesbezüglich vorteilhafte Ausgestaltungen sind in der EP 3 922 217 A1 beschrieben, auf die hiermit vollinhaltlich Bezug genommen wird.

Der medizinische Wirkstoff kann vorzugsweise in ein Polymer eingelagert sein, sodass die medizinische Wirkstoffschicht ausgebildet wird, und wobei der medizinische Wirkstoff vorzugsweise ein Antiproliferativum, bspw. Sirolimus, oder andere Limus-Derivate oder Paclitaxel (PTX) oder ein langzeitstabiles Depot-Gestagen, bspw. Etonogestrel, Levonorgestrel oder ein Antiprogesteron, bspw. Mifepriston oder ein Spermizid bspw. Nonoxinol 9 oder ein Zytostatikum, bspw. Mitomycin, Capecitabin oder Methotrexat (MTX) ist.

Die Funktionshülse kann an einem proximalen und/oder an einem distalen Ende einen geringeren Querschnitt aufweisen als der Rest der Hülse, wobei vorzugsweise ein proximaler und/oder ein distaler sich in etwa konusförmiger verjüngender Abschnitt vorgesehen sind.

Durch die Querschnittsreduzierung der Funktionshülse kann diese gegen ein Verrutschen auf dem Ballon gesichert werden.

Herkömmliche Ballons tendieren beim Entfalten zu einem verstärkten Aufdehnen in ihren distalen und proximalen Abschnitten ("Dog-Bone"), wodurch die Gefäßwand dort besonders traumatisiert werden kann. Bei den Ausführungsformen der erfindungsgemäßen Ballonkathetervorrichtung ist es daher vorteilhaft, auf fenster- oder schlitzförmige Durchgangsöffnungen der Funktionshülse in den unmittelbar den Ballonschultern benachbarten Abschnitten proximal und distal zu verzichten, um eine Dog-Bone-Deformation des Ballons beim Entfalten zu verhindern.

Weiterhin kann die Funktionshülse über proximale und/oder distale Laschenelemente mit dem Katheterschaft des Ballonkatheters verbunden sein.

Dies bewirkt ebenfalls eine Sicherung gegen Verrutschen und insbesondere erleichtert dies das Einziehen der Hülse in den Ballonkatheter.

Die rohrförmige Funktionshülse kann eine tangentiale und/oder eine axiale Stützschicht aufweisen, wobei die tangentiale Stützschicht durch Polymer-Nano-Fasern mit höherer Festigkeit und/oder durch eine zusätzliche Polymerschicht ausgebildet ist (z.B. durch ein laser-geschnittenes tubuläres, degradierbares Polymer-Halbzeug, oder durch eine mittels Melt-Electrospinning-Writing-MEW gebildete Schicht) und/oder dass die Funktionshülse tangential umlaufend und gleich beabstandet voneinander angeordnete axiale Stützstreben aufweist, die vorzugsweise im Bereich der inneren Faltungslinien außerhalb der Funktionshülse angeordnet sind.

Durch eine derartige bauliche Ausgestaltung lässt sich die Steifigkeit der Funktionshülse in axialer und tangentialer Richtung einstellen bzw. verbessern.

Die Beschichtung der Ballonmembran durch Dip-Coating oder als aufgesprühtes Polymer-Vlies oder als ummantelnde Zwischenhülse kann adhäsive Eigenschaften aufweisen.

Durch die adhäsiven Eigenschaften der wirkstoffenthaltenden Polymerkomponente wird das Anhaften an der Hohlorganwand begünstigt. Das bedeutet konkret, dass die über die Durchgangsöffnung der Funktionshülse abgepressten Anteile der medizinischen Wirkstoffschicht beim Entfalten an der Innenwandung eines Hohlorgans anhaften.

Die Hülse kann mit der Ballonmembran stoffschlüssig verbunden sein, z.B. durch punktuelles Kleben oder Polymer-Schweißen, wobei die Verbindungspunkte bevorzugt auf inneren Faltungslinien der Ballonmembran angeordnet sind.

Die Funktionshülse ist vorzugsweise aus einem gering dehnfähigen Polymer ausgebildet, dessen Dehnfähigkeit geringer als die Dehnfähigkeit der Ballonmembran des Ballons ist.

Eine Gesamtfläche der Durchgangsöffnung, wenn diese als Schlitze ausgebildet sind, kann zumindest 10 Prozent bzw. zumindest 15 Prozent bzw. zumindest 20 Prozent bis maximal 50 Prozent bzw. 45 Prozent bzw. 40 Prozent bzw. maximal 35 Prozent und insbesondere maximal 30 Prozent der Gesamtfläche der Funktionshülse betragen, oder
eine Gesamtfläche der Durchgangsöffnungen kann, wenn diese als fensterartige Öffnungen ausgebildet sind, zumindest 30 Prozent bzw. zumindest 35 Prozent bzw. zumindest 40 Prozent bzw. zumindest 45 Prozent bzw. zumindest 50 Prozent bis 85 Prozent bzw. maximal 80 Prozent bzw. 75 Prozent bzw. maximal 70 Prozent der Gesamtfläche der Funktionshülse betragen.

Zudem kann auf der Funktionshülse im Ausgangszustand eine äußere Schutzhülle angeordnet sein.

Durch eine solche folienartige Schutzhülle wird ein Blutkontakt vermieden und somit eine Thrombusbildung während des Einbringens der Hülse verhindert. Am Implantationsort kann die Schutzhülle dann durch Zurückziehen oder durch ein entfaltungsbedingtes Zerreißen entfernt werden. Darüber hinaus kann die Stabilität der Faltung der Hülse für den Transport auf dem Ballonkatheter durch eine adhäsive Oberflächenbehandlung der Außenfläche der Hülse unterstützt werden.

Weiterhin ist erfindungsgemäß ein Verfahren zur Herstellung einer Funktionshülse für eine vorstehend aufgezeigte Ballonkathetervorrichtung vorgesehen. Dieses umfasst die folgenden Schritte:
Blasformen eines rohrförmigen Polymer-Rohlings in einer temperierten metallischen Form auf den Durchmesser einer inneren Mantelwandung,
Entformung und Ablängung im Bereich von Hülsenschultern,
Aufziehen der Hülse auf einen Haltekern, wobei dieser auch aus einem entfaltbaren Drahtgeflecht bestehen kann, das sich sowohl an den Hülsen-Entfaltungsdurchmesser als auch an den geringeren Durchmesser der Hülsenschultern anpassen kann,
Schneiden, insbesondere Laser-Schneiden von Durchgangsöffnungen in die Hülse, um die Funktionshülse auszubilden.

Zudem ist erfindungsgemäß ein Verfahren zur Herstellung einer vorstehend aufgezeigten Ballonkathetervorrichtung vorgesehen. Dieses umfasst die folgenden Schritte:
Aufbringen einer medizinischen Wirkstoffschicht auf eine äußere Mantelwandung eines vorzugsweise vorgefalteten Ballons einer Ballonkathetervorrichtung im aufgedehnten Zustand des Ballons,
Aufbringen (axiales Aufziehen) einer vorgefalteten Funktionshülse auf/über die getrocknete Wirkstoffschicht bei deflatiertem Ballon, wobei die Orientierung derart ist, dass die Falten von Ballon und Hülse ineinander liegen, Verbinden der Funktionshülse und des Ballons (chemische, stoffschlüssige Verbindung: Schweißen, Kleben),
gleichmäßiges Falten und Wickeln des Ballons gemeinsam mit der Funktionshülse um eine Längsachse der Ballonkathetervorrichtung zur Lagerung bis zur Anwendung.

Das Auftragen der Polymer-Gel-Lösung auf den Ballon oder auf einen zylindrischen Formkörper (separates Vlies) kann durch Versprühen mit einem Luftstrahl (Airspraying) oder durch Verspinnen in einem elektrischen Feld (Electrospinning) und/oder durch eine Kombination davon (Electrostatic Airspraying) und/oder durch Tauchen in eine Lösung (Dip-Coating) und/oder durch Aufbringen eines kontinuierlichen Schmelzestrangs (Melt-Electrospinning-Writing) erfolgen oder diskontinuierlich mittels 3D-Druck aufgetragen werden.

Zudem kann auf die Ballonmembran oder auf den Träger des Vlieses vor dem Auftragen des Gemisches eine Trennschicht aufgetragen werden.

Das Vorsehen einer entsprechenden Trennschicht erleichtert später das Lösen der Wirkstoffschicht von der Ballonmembran bzw. vom Träger.

Die Vorteile der erfindungsgemäßen Verfahren entsprechen analog den Vorteilen, die vorstehend anhand der erfindungsgemäßen Ballonkathetervorrichtung erläutert wurden.

Für alle Anwendungen der erfindungsgemäßen Ballonkathetervorrichtung in Hohlorganen gilt dass damit eine atraumatische Aufdehnung von Engstellen durch die kissenförmige Segmentierung der Außenkontur erzielt werden kann. Mögliche Wirkstoff-Applikationen lassen sich je nach Hohlorgan-Anwendung wie folgt kurz zusammenfassen:
- Für den Einsatz im Blutgefäß eignen sich Antiproliferativa wie Limus-Derivate (z.B. Sirolimus) oder Paclitaxel (PTX) als Wirkstoff für die Übertragung in die Gefäßwand zur Reduzierung einer überschießenden Wandreaktion (intimale Hyperplasie)
- Bei einer Applikation im Eileiter eignen sich Wirkstoffe mit kontrazeptiver Wirkung z. B. langzeitstabile Depot-Gestagene, wie z.B. Etonogestrel, Levonorgestrel oder ein geeignetes Antiprogesteron, wie z.B. Mifepriston oder ein Spermizid, wie z.B. Nonoxinol 9 vorgesehen sein. Grundsätzlich ist auch eine Applikation mit einem kontrazeptiven Wirkstoff im Bereich des Ductus deferens (Samenleiter) möglich.
- Zur Therapie von Karzinomen in Hohlorganen, z.B. in den Gallenwegen, können als medizinsche Wirkstoffe Zytostatika wie z.B. Mitomycin, Capecitabin oder Methotrexat (MTX) vorgesehen sein.
- Weitere mögliche Applikationen umfassen weitere Hohlorgane wie den Pankreasgang, die ableitenden Harnwege, Lymphgefäße, wie den Ductus thoracicus, das Tracheobronchialsystem, den Ductus nasolacrimalis, die Tuba Eustachii oder auch den Gastrointestinaltrakt.

Weiterhin sind medizinische und therapeutische Verfahren zur Behandlung von Hohlorganen mit der erfindungsgemäßen Ballonkathetervorrichtung vorgesehen.

Für den Einsatz im Blutgefäß soll die erfindungsgemäßen Ballonkathetervorrichtung als Träger für Antiproliferativa wie Limus-Derivate dienen und damit deren Übertragung mit einem längerfristigen Kontakt zur Gefäßwand gewährleisten.

Bei der Anwendung in Blutgefäßen haben Limus-Derivate eine viel günstigere biologische Wirkung als Paclitaxel. Sie lassen sich jedoch schlecht mit herkömmlich beschichteten Ballons per einmaligem kurzen Ballonkontakt in die Gefäßwand übertragen, da sie deutlich schlechter anhaften als Paclitaxel-Kristalle, die in die Gefäßwand gespießt werden. Mit der erfindungsgemäßen Ballonkathetervorrichtung wird die Polymer-Wirkstoffschicht über die Durchgangsöffnungen der Funktionshülse als "Gel-Spots" oder Micro-Flakes" an bzw. in die Gefäßwand gepresst.

Bei einer Applikation der erfindungsgemäßen Ballonkathetervorrichtung im Bereich der Eileiter können Wirkstoffe mit kontrazeptiver Wirkung wie langzeitstabile Depot-Gestagene, z.B. Etonogestrel, Levonorgestrel oder ein geeignetes Antiprogesteron, wie z.B. Mifepriston oder ein Spermizid, wie z.B. Nonoxinol 9 vorgesehen sein. Grundsätzlich ist auch eine Applikation mit einem kontrazeptiven Wirkstoff im Bereich des Ductus deferens (Samenleiter) möglich.

Zur Therapie von Karzinomen in Hohlorganen, z.B. in den Gallenwegen, können für die erfindungsgemäße Ballonkathetervorrichtung als medizinsche Wirkstoffe Zytostatika wie z.B. Mitomycin, Capecitabin oder Methotrexat (MTX) vorgesehen sein.

Weitere mögliche Applikationen der Ballonkathetervorrichtung umfassen weitere Hohlorgane wie den Pankreasgang, die ableitenden Harnwege, Lymphgefäße, wie den Ductus thoracicus, das Tracheobronchialsystem, den Ductus nasolacrimalis, die Tuba Eustachii oder auch den Gastrointestinaltrakt.

Die vorliegende Erfindung wird im Folgenden anhand mehrerer in den Figuren dargestellten Ausführungsbeispiele beschrieben. Diese zeigen in
Figur 1 eine schematische seitliche Darstellung einer erfindungsgemäßen Ballonkathetervorrichtung gemäß einem ersten Ausführungsbeispiel, mit fensterartig ausgebildeten Durchgangsöffnungen,
Figur 2 eine schematische seitliche Darstellung der erfindungsgemäßen Ballonkathetervorrichtung gemäß einem zweiten Ausführungsbeispiel, mit fensterartig ausgebildeten Durchgangsöffnungen und sich konusförmig verjüngenden Abschnitten am proximalen und am distalen Ende,
Figur 3 eine schematische seitliche Darstellung der erfindungsgemäßen Ballonkathetervorrichtung gemäß einem dritten Ausführungsbeispiel, mit dreieckigen fensterartigen Durchgangsöffnungen,
Figur 4 eine schematische seitliche Darstellung der erfindungsgemäßen Ballonkathetervorrichtung gemäß einem vierten Ausführungsbeispiel, mit drei Schlitzreihen aus sich in Längsrichtung erstreckenden Axialschlitzen und proximalen sowie distalen Laschenelementen,
Figur 5 eine schematische seitliche Darstellung der erfindungsgemäßen Ballonkathetervorrichtung gemäß einem fünften Ausführungsbeispiel, mit drei Schlitzreihen und einem Muster aus Poren (Perforationen) (nicht dargestellt) und sich konusförmig verjüngenden Abschnitten am proximalen und am distalen Ende,
Figur 6 eine schematische seitliche Darstellung der erfindungsgemäßen Ballonkathetervorrichtung gemäß einem sechsten Ausführungsbeispiel, mit drei Schlitzreihen, sich konusförmig verjüngenden Abschnitten am proximalen und am distalen Ende und sich in Axialrichtung erstreckenden Stützstreben,
Figur 7 eine schematische seitliche Darstellung der erfindungsgemäßen Ballonkathetervorrichtung gemäß einem sechsten Ausführungsbeispiel mit einer segmentierten Struktur aus miteinander verbundenen Umfangsringen,
Figur 8 eine schematische Darstellung eines Faltmusters (Plissierung in 3 Falten) der Funktionshülse,
Figur 9 eine schematische Darstellung eines Drittels eines Umfangssegments, entsprechend der Mantelfläche einer Falte, im abgewickelten Zustand,
Figur 10 eine weitere Ausführungsform eines Drittels eines Umfangssegments, entsprechend der Mantelfläche einer Falte, im abgewickelten Zustand,
Figur 11 eine schematische Darstellung eines Funktionshülsenabschnitts mit axial orientierten Schlitzen einer Funktionshülse, das eine deutliche kissenartige Vorwölbung einer darunterliegenden Ballonmembran bei Aufdehnung erlaubt,
Figur 12 eine Darstellung eines Funktionshülsenabschnitts mit tangential orientierten Schlitzen einer Funktionshülse, das eine geringe Vorwölbung einer darunterliegenden Ballonmembran bei Aufdehnung erlaubt,
Figur 13 eine weitere Darstellung eines abschnittweisen Schlitzmusters von Schlitzen einer Funktionshülse,
Figur 14 eine weitere Darstellung eines abschnittweisen Schlitzmusters von Schlitzen einer Funktionshülse,
Figur 15 eine schematische Darstellung von gruppierten Axialschlitzen und Löchern (Poren) der Funktionshülse,
Figur 16 eine schematische Darstellung von einer Kombination aus gruppierten Axialschlitzen und Löchern mit gewollter Rissbildung zwischen jeweils axial eng benachbarten Schlitzen bei Ballonaufdehnung,
Figur 17 ein schematische Draufsicht auf eine vorteilhafte Ausführungsform der Funktionshülse der erfindungsgemäßen Ballonkathetervorrichtung, und
Figur 18 eine schematische Darstellung einer erfindungsgemäßen Ballonkathetervorrichtung mit einer Funktionshülse gemäß Figur 17.

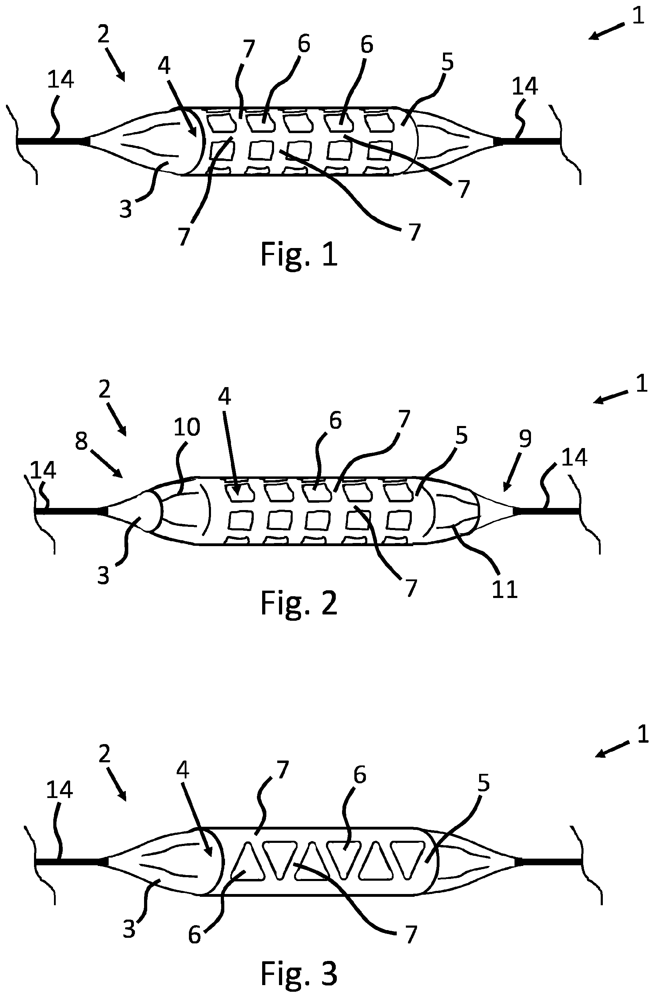

Eine erfindungsgemäße Ballonkathetervorrichtung 1 wird im Folgenden anhand eines ersten Ausführungsbeispiels näher beschrieben (Figur 1).

Die Ballonkathetervorrichtung 1 ist zur atraumatischen Behandlung von Hohlorganen ausgebildet und umfasst einen Ballonkatheter 2 (ohne proximalen Katheterschaft dargestellt) mit einem Ballon 3. Auf eine äußere Mantelwandung des Ballons 3 ist eine medizinische Wirkstoffschicht 4 aufgebracht.

Die medizinische Wirkstoffschicht 4 ist als eine Beschichtung einer Ballonmembran des Ballons 3 ausgebildet, wobei der medizinische Wirkstoff in einer Polymer-Gel-Formulierung enthalten ist.

Alternativ kann die medizinische Wirkstoffschicht 4 als ein auf der Ballonmembran aufgesprühtes biodegradierbares Polymer-Vlies mit Kompartimenten bzw. Mikrokompartimenten ausgebildet sein, in die der medizinische Wirkstoff eingelagert ist.

Weiter alternativ kann die medizinische Wirkstoffschicht 4 als rohrförmige Zwischenhülse mit einer Vliesstruktur aus biodegradierbaren Polymer mit Mikrokompartimenten bzw. Kompartimenten, in die der medizinische Wirkstoff eingelagert ist, ausgebildet sein.

Auf der medizinischen Wirkstoffschicht 4 bzw. auf dem Ballon 3 des Ballonkatheters 2 ist eine Funktionshülse 5 angeordnet, die mit dem Ballon 3 des Ballonkatheters 2 verbunden ist.

Die Verbindung der Hülse 5 ist insbesondere stoffschlüssig mit der Ballonmembran bzw. dem Ballon 3 verbunden. Die Verbindung erfolgt z.B. durch punktuelles Schweißen, flächiges Kleben oder durch großflächige Adhärenz eines wirkstoffhaltigen Gels zwischen Ballonmembran und Funktionshülse. Punktuelle oder linienförmige Verbindungen sind bevorzugt entlang von inneren Faltungslinien angeordnet.

Gemäß dem ersten Ausführungsbeispiel ist die Funktionshülse 5 in etwa rohrförmig bzw. zylindrisch aus einem gering dehnfähigen Polymer ausgebildet.

In der Funktionshülse 5 sind in etwa rechteckförmige Durchgangsöffnungen 6 vorgesehen, die durch ebenflächige Abschnitte bzw. ebenflächige Streben 7 begrenzt sind.

Hierbei ist vorgesehen, dass in etwa die gesamte Fläche der Funktionshülse 5 die Durchgangsöffnung 6 aufweist bzw. die Durchgangsöffnung 6 über nahezu die gesamte Fläche der Funktionshülse 5 angeordnet sind.

In einem Ausgangszustand ist die Funktionshülse 5 zusammen mit der medizinischen Wirkstoffschicht 4 und den Ballon 3 um eine Hülsenlängsachse gefaltet, wobei die Faltung als Plissierung um eine Hülsenlängsachse gerichtet ist und in einem Endzustand zum Anliegen an einer Innenwandung eines Hohlorgans entfaltbar ist.

Die Durchgangsöffnungen weisen gemäß allen Ausführungsbeispielen abgerundete Ecken auf.

Weiterhin weist die Funktionshülse 5 im Bereich ihrer Streben 7 bzw. auf ihre gesamten Mantelwandung Poren 20 (nicht dargestellt in Figur 1) auf.

Im Folgenden wird die erfindungsgemäße Ballonkathetervorrichtung 1 anhand eines zweiten Ausführungsbeispiels näher beschrieben (Figur 2). Sofern nichts anderes beschrieben ist, entspricht das zweite Ausführungsbeispiel genauso wie die weiteren noch folgenden Ausführungsbeispiele im Wesentlichen der Ballonkathetervorrichtung 1 gemäß dem ersten Ausführungsbeispiel. Gleiche technische Merkmale sind mit den gleichen Bezugszeichen versehen.

Gemäß dem zweiten Ausführungsbeispiel weist die Funktionshülse 5 der Ballonkathetervorrichtung 1 an einem proximalen und an einem distalen Ende 8, 9 einen geringeren Querschnitt auf als der Rest der Funktionshülse. Diese Abschnitte werden als proximaler und distaler konusförmiger Abschnitt 10, 11 bezeichnet. Der proximale konusförmige Abschnitt 10 und der distale konusförmige Abschnitt 11 sind ausgebildet, um ein axiale Verschiebung der Funktionshülse 5 auf dem Ballon 3 zu verhindern.

Gemäß einem dritten Ausführungsbeispiel sind die Durchgangsöffnungen 6 in etwa dreieckförmig ausgebildet (Figur 3).

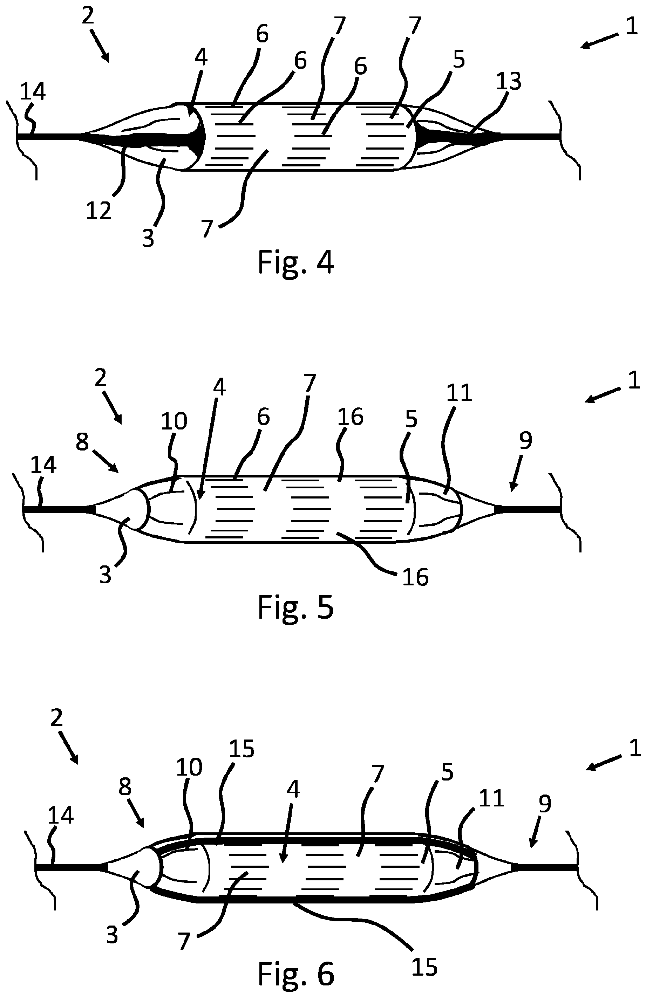

Gemäß einem vierten Ausführungsbeispiel sind die Durchgangsöffnungen 6 als sich in Axialrichtung erstreckende Schlitze ausgebildet, wobei im vorliegenden Ausführungsbeispiel drei Reihen von tangential umlaufenden axialen Schlitzen vorgesehen sind (Figur 4).

Zudem ist die Funktionshülse 5 gemäß dem vierten Ausführungsbeispiel der Ballonkathetervorrichtung über proximale und distale Laschenelemente 12, 13 mit einem Katheterschaft 14 des Ballonkatheters 2 verbunden.

Auf diese Weise wird die Funktionshülse 5 gegen ein Verrutschen auf dem Ballon 3 der Ballonkatheter-vorrichtung 1 gesichert. Zudem erleichtern die Laschenelemente 12, 13 das Einziehen der Funktionshülse 5 zusammen mit dem Ballonkatheter 2 in einen Führungskatheter oder eine Schleuse.

Gemäß einem fünften Ausführungsbeispiel, das im Wesentlichen dem vierten Ausführungsbeispiel entspricht, sind die Durchgangsöffnungen 6 ebenfalls in Form von Axialschlitzen ausgebildet, wobei die Funktionshülse zudem den proximalen konusförmigen Abschnitt 10 und den distalen konusförmigen Abschnitt 11 umfasst und im Bereich der Streben 7 ein Muster von Poren (Perforationen; nicht dargestellt) aufweist (Figur 5).

Gemäß einem sechsten Ausführungsbeispiel, das im Wesentlichen dem fünften Ausführungsbeispiel entspricht, weist die Funktionshülse 5 drei Axialstreben 15 auf, die sich über in etwa die gesamte Länge der Funktionshülse 5 in Axialrichtung erstrecken und tangential umlaufend in etwa gleich beabstandet voneinander in einem Winkel von in etwa 120° angeordnet sind (Figur 6). Gemäß diesem Ausführungsbeispiel sind drei axiale Stützstreben vorgesehen, um die Steifigkeit der Funktionshülse in axialer Richtung zu erhöhen. Es können aber auch zwei oder vier oder fünf oder mehr Axialstreben 15 vorgesehen sein.

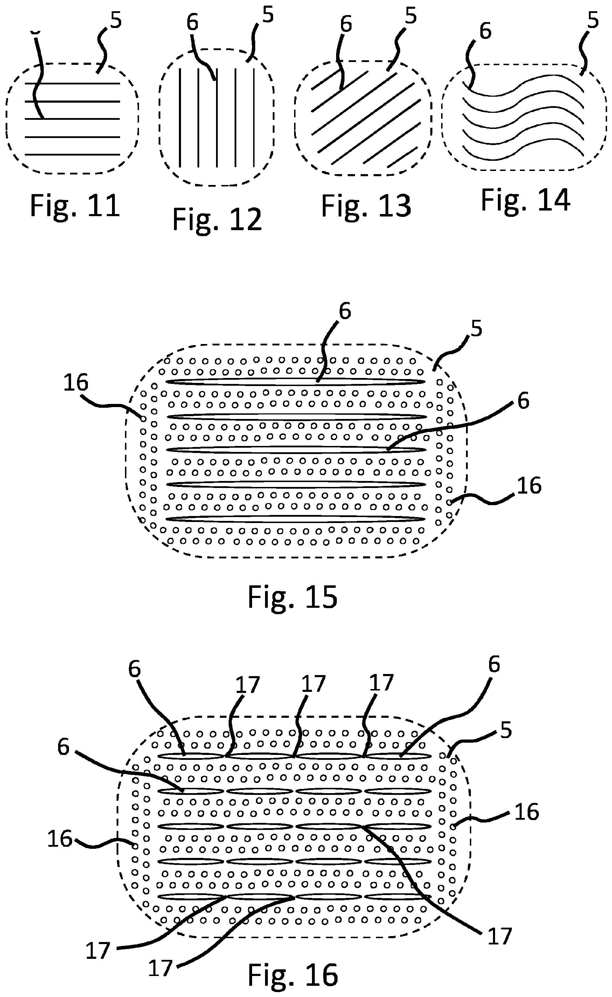

Die als Schlitze ausgebildeten Durchgangsöffnungen gemäß dem vierten, fünften und sechsten Ausführungsbeispiel können in Axialrichtung über Sollreißstellen 17 (Fig. 16) miteinander verbunden sein, sodass bei einer Ballonaufdehnung mehrere benachbarte axiale Schlitze sich zu einem gemeinsamen axialen Schlitz verbinden und eine größere Kissenbildung in diesem Bereich ermöglichen.

Gemäß einem siebten Ausführungsbeispiel sind die Durchgangsöffnungen 6 der Funktionshülse in Form von tangential umlaufenden Ausnehmungen bzw. Schlitzen ausgebildet, die jedoch zumindest einmal von axial verlaufenden Streben 7 unterbrochen sind.

Wie vorstehend aufgezeigt, können die technischen Merkmale der vorliegenden Erfindung hinsichtlich der geometrischen bzw. baulichen Ausgestaltung der Durchgangsöffnungen 6, der Streben 7, der konusförmigen Abschnitte 10, 11, der Laschenelemente 12, 13, der Axialstreben oder Tangentialstreben oder auch einer axialen oder einer tangentialen Stützschicht und der Sollreißstellen 17, sofern technisch möglich und sinnvoll, beliebig miteinander kombiniert werden.

Verschiedene bzw. unterschiedliche Ausgestaltungen der Durchgangsöffnungen 6, sofern diese als Schlitze ausgebildet sind, sind in den Figuren 11 bis 16 dargestellt.

Vorzugsweise erstrecken sich die Schlitze lediglich in Axialrichtung (Figur 11).

Die Schlitze können jedoch auch gegenüber der Axialrichtung geneigt sein (Figur 13).

Zudem können die Schlitze auch wellenförmig ausgebildet sein (Figur 14).

Weiterhin können sich die Schlitze auch gemäß einer nicht vorteilhaften Ausgestaltung in Tangentialrichtung erstrecken, woraus keine wesentliche Kissenbildung einer darunter befindlichen, aufgedehnten Ballonmembran resultiert (Figur 12).

Im Bereich der Streben 7 und insbesondere auch im Bereich zwischen den Axialschlitzen sind Poren 16 über die gesamte Mantelwandung der Funktionshülse 5 verteilt angeordnet (Figur 15).

Gemäß einer solchen Ausgestaltung können die Schlitze auch kürzer ausgebildet sein, wobei dann beispielsweise vier Axialschlitze über Sollreißstellen 17 beim Expandieren des Ballons 3 miteinander verbindbar sind (Figur 16).

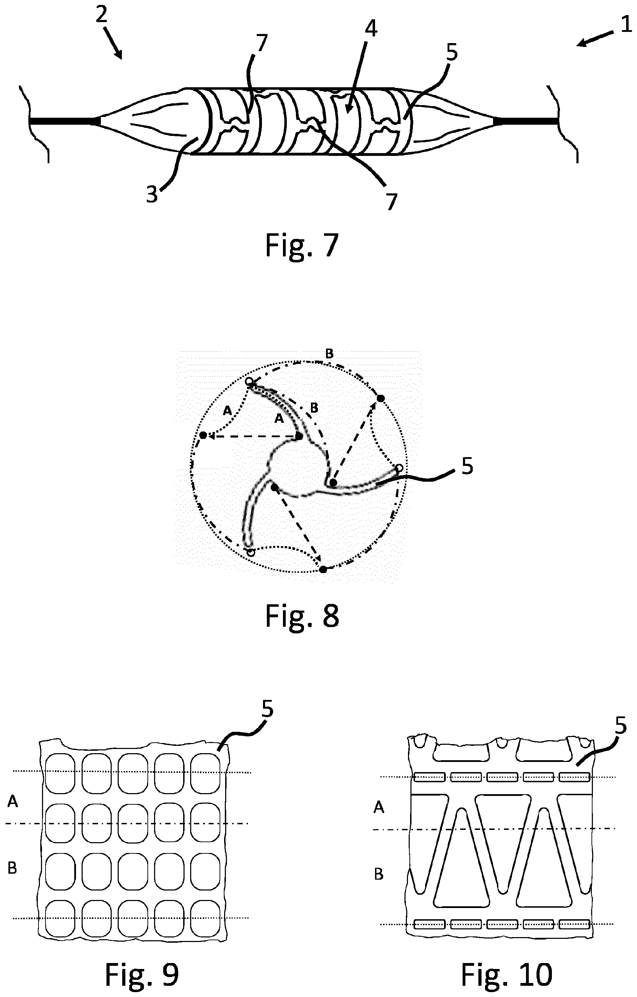

Der Ballon 3, die medizinische Wirkstoffschicht 4 und die Funktionshülse 5 der Ballonkathetervorrichtung 1 sind in einem Ausgangszustand um eine Katheterlängsachse gefaltet bzw. plissiert. Bei der Faltung sind beispielsweise drei gleich große auf dem Umfang gleichmäßige verteilte in Axialrichtung verlaufende Falten vorgesehen. Die Falten erstrecken sich entlang einer ersten Faltungslinie A und einer zweiten Faltungslinie B (Figuren 8 bis 10).

Die Falten sind gleichmäßig und gleichsinnig um die Längsachse des Ballonkatheters gewunden und an diesen angelegt.

Weiterhin ist erfindungsgemäß ein Verfahren zur Herstellung einer Funktionshülse für eine vorstehend aufgezeigte Ballonkathetervorrichtung vorgesehen. Dieses umfasst die folgenden Schritte:
Blasformen eines rohrförmigen Polymer-Rohlings in einer temperierten metallischen Form auf den Durchmesser einer inneren Mantelwandung,
Entformung und Ablängung im Bereich von Hülsenschultern,
Aufziehen der Hülse auf einen Haltekern, wobei dieser auch aus einem entfaltbaren Drahtgeflecht bestehen kann, dass sich sowohl an den Hülsen-Entfaltungsdurchmesser als auch an den geringeren Durchmesser der Hülsenschultern anpassen kann,
Schneiden, insbesondere Laser-Schneiden von Durchgangsöffnungen in die Hülse, um die Funktionshülse auszubilden.

Zudem ist erfindungsgemäß ein Verfahren zur Herstellung einer vorstehend aufgezeigten Ballonkathetervorrichtung vorgesehen. Dieses umfasst die folgenden Schritte:
Aufbringen einer medizinischen Wirkstoffschicht auf eine äußere Mantelwandung eines vorzugsweise vorgefalteten Ballons einer Ballonkathetervorrichtung im aufgedehnten Zustand des Ballons,
Aufbringen (axiales Aufziehen) einer vorgefalteten Funktionshülse auf/über die getrocknete Wirkstoffschicht bei deflatiertem Ballon, wobei die Orientierung derart ist, dass die Falten von Ballon und Hülse ineinander liegen, Verbinden der Funktionshülse und des Ballons (Schweißen, Kleben, adhäsive Wirkstoffschicht),
gleichmäßiges Falten und Wickeln des Ballons gemeinsam mit der Funktionshülse um eine Längsachse der Ballonkathetervorrichtung zur Lagerung bis zur Anwendung und für den Transport zum Wirkort.

Das Auftragen der Polymer-Gel-Lösung auf den Ballon oder auf einen zylindrischen Formkörper (separates Vlies) kann durch Versprühen mit einem Luftstrahl (Airspraying) oder durch Verspinnen in einem elektrischen Feld (Electrospinning) und/oder durch eine Kombination davon (Electrostatic Air-spraying) und/oder durch Tauchen in eine Lösung (Dip-Coating) und/oder durch Aufbringen eines kontinuierlichen Schmelzestrangs (Melt-Electrospinning-Writing) erfolgen oder diskontinuierlich mittels 3D-Druck aufgetragen werden.

Zudem kann auf die Ballonmembran oder auf den Träger des Vlieses vor dem Auftragen des Gemisches eine Trennschicht aufgetragen werden.

Das Vorsehen einer entsprechenden Trennschicht erleichtert später das Lösen der Wirkstoffschicht von der Ballonmembran bzw. des Vlieses vom Träger.

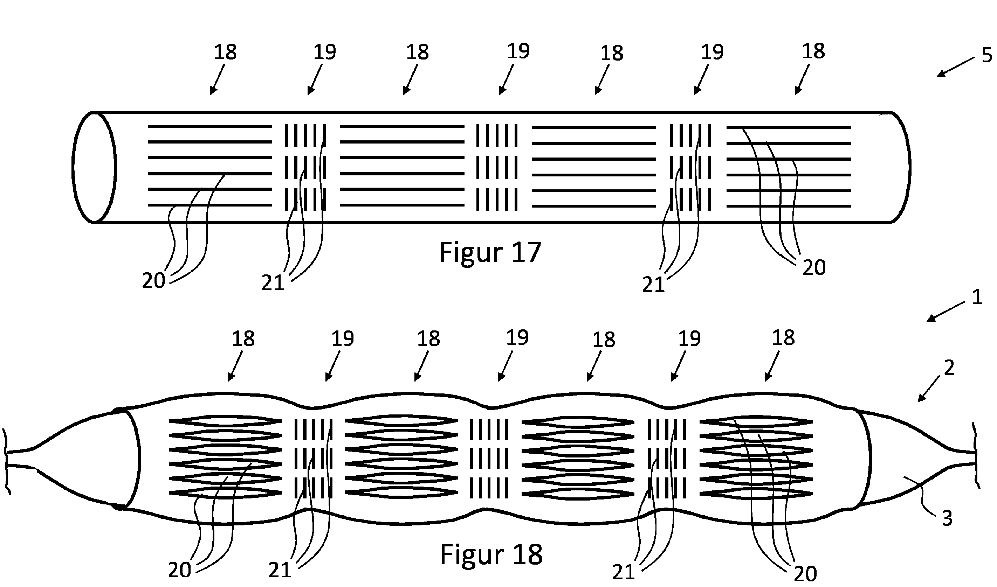

Im Folgenden wird eine bevorzugte Ausführungsform einer erfindungsgemäßen Ballonkathetervorrichtung 1 zur schonenden kissenartigen Ballonaufdehnung des Hohlorgans und gleichzeitiger Wirkstoffapplikation in die Hohlorganwand näher beschrieben (Figur 17). Sofern nichts anderes beschrieben ist, weist diese Ballonkathetervorrichtung 1 sämtliche Merkmale der vorstehend beschriebenen Ballonkathetervorrichtungen auf. Gleiche technische Merkmale sind mit den gleichen Bezugszeichen versehen.

Diese Ballonkathetervorrichtung 1 umfasst den Ballonkatheter 2 mit dem Ballon 3. Auf der äußeren Mantelwandung des Ballons 3 ist eine medizinische Wirkstoffschicht 4 aufgebracht.

Weiterhin ist auf dem Ballonkatheter 2 die Funktionshülse 5 angeordnet, wobei die Funktionshülse 5 mit dem Ballonkatheter 2 im Wesentlichen über ihre gesamte Länge verbunden ist. Hierbei kann eine verteilt punktuelle, eine flächige oder eine im Wesentlichen flächige Verbindung vorgesehen sein.

Bevorzugt ist jedoch eine Verbindung entlang von Verbindungspunkten oder -linien der Schweißbahnen, hergestellt durch Kunststoff-, insbesondere Polymerschweißen. Entsprechende Verbindungspunkte oder -linien liegen bevorzugt auf Faltungslinien der Hülse und des Ballons, insbesondere auf inneren Faltungslinien.

Die Funktionshülse 5 weist eine höhere Steifigkeit auf als der Ballon 3, bzw. bei annähemd gleicher Wanddicke ein höheres Elastizitätsmodul.

In der Funktionshülse 5 sind in Längsrichtung alternierend Axialabschnitte 18 und Tangentialabschnitte 19 beabstandet zueinander angeordnet.

An einem proximalen Ende der Funktionshülse 5 ist zunächst einer der Axialabschnitte 18 ausgebildet.

Im Axialabschnitt 18 sind tangential bzw. radial umlaufend und in etwa gleich beabstandet voneinander sich in Axialrichtung erstreckende Axialschlitze 20 ausgebildet. Die Axialschlitze 20 weisen in Axialrichtung in etwa die gleiche Länge auf und sind in tangentialer Richtung fluchtend zueinander angeordnet.

An den ersten Axialabschnitt 18 schließt sich ein erster Tangentialabschnitt 19 an.

Die Axialabschnitte 18 sind beabstandet zu den Tangentialabschnitten 19 in Längsrichtung zueinander angeordnet.

Im Tangentialabschnitt 19 sind mehrere sich quer zur Längsrichtung erstreckende bzw. in Tangentialrichtung erstreckende Tangentialschlitze 21 ausgebildet. Die Tangentialschlitze 21 sind in Axialrichtung gleich beabstandet zueinander angeordnet und erstrecken sich tangential bzw. radial umlaufend über die Mantelwandung der Funktionshülse 5.

Der Ballon 3 des Ballonkatheters 2 sowie die mit diesem verbundene Funktionshülse 5 sind um eine Längsachse der Ballonkatheter-Vorrichtung 1 gemeinsam gefaltet und plissiert.

Zur atraumatischen Behandlung von Hohlorganen ist dann vorgesehen, die Ballonkatheter-Vorrichtung 1 im Bereich einer zu behandelnden Stelle eines Hohlorgans anzuordnen.

Dann wird der Ballon 3 des Ballonkatheters 2 expandiert, so dass sich der Ballon 3 zusammen mit der damit verbundenen Funktionshülse 5 entfaltet.

Im Anschluss daran erfolgt eine Aufweitung des Hohlorgans mit Kissenbildung der Ballonmembran des Ballons 3 im Bereich der Tangentialschlitze 21. Im Bereich der Axialschlitze 20 und der Tangentialschlitze 21 erfolgt dann eine Wirkstoffapplikation auf eine innere Mantelwandung des Hohlorgans.

Nach Beendigung der Hohlorganaufweitung und Wirkstoffapplikation wird der Ballon 3 des Ballonkatheters 2 deflatiert, wobei sich dabei der Ballon 3 und die Funktionshülse 5 entlang vorbestimmter Faltungslinien, die zuvor beim Falten und Plissieren entstanden sind, wieder zusammenfalten. Anschließend kann die Ballonkatheter-Vorrichtung 1 aus dem menschlichen Körper entfernt werden.

Hierbei kann auch vorgesehen sein, dass die Funktionshülse 5 über ihre im Wesentlichen gesamte Fläche Poren aufweist. Durch diese Poren kann ebenfalls der medizinische Wirkstoff appliziert werden.

Gemäß vorliegender Ausführungsform ist vorgesehen, dass an einem proximalen und an einem distalen Ende der Funktionshülse 5 Axialabschnitte 18 angeordnet sind. Insgesamt sind gemäß dieser Ausführungsform vier Axialabschnitte 18 vorgesehen, wobei zwischen den Axialabschnitten 18 und jeweils beabstandet zu diesen entsprechend drei Tangentialabschnitte 19 angeordnet sind.

Sämtliche Ausführungsformen und Ausführungsbeispiele der erfindungsgemäßen Ballonkathetervorrichtung sind sofern technisch sinnvoll und möglich beliebig miteinander kombinierbar.

### Bezugszeichenliste

- 1: Ballonkathetervorrichtung
- 2: Ballonkatheter
- 3: Ballon
- 4: Medizinische Wirkstoffschicht
- 5: Funktionshülse
- 6: Durchgangsöffnungen
- 7: Streben
- 8: Proximales Ende
- 9: Distales Ende
- 10: Proximaler konusförmiger Abschnitt
- 11: Distaler konusförmiger Abschnitt
- 12: Proximale Laschenelemente
- 13: Distale Laschenelemente
- 14: Katheterschaft
- 15: Axialstreben
- 16: Poren
- 17: Sollreißstellen
- 18: Axialabschnitt
- 19: Tangentialabschnitt
- 20: Axialschlitz
- 21: Tangentialschlitz

## Patentansprüche

1. Ballonkathetervorrichtung (1) zur atraumatischen Aufdehnung von Hohlorganen, umfassend einen Ballonkatheter (2) mit einem Ballon (3),
eine auf dem Ballon (3) applizierte rohrförmige Funktionshülse (5) zur gerichteten segmentierten kissenartigen Entfaltung des Ballons (3),
wobei die Funktionshülse (5) in einem Ausgangszustand zusammen mit dem Ballon (3) um eine Hülsenlängsachse gefaltet ist, wobei die Faltung der Funktionshülse (5) als Plissierung um eine Hülsenlängsachse gerichtet ist und in einem Endzustand zum Anliegen an einer Innenwandung eines Hohlorganes entfaltbar ist, und wobei die rohrförmige Funktionshülse (5) ebenflächige Abschnitte und/oder ebenflächige Streben (7) aufweist, die Durchgangsöffnungen (6) begrenzen, **dadurch gekennzeichnet, dass** die Funktionshülse (5) zur abschnittweisen Begrenzung einer Ballonaufdehnung des Ballons (3) ausgebildet ist, wobei die Funktionshülse (5) im Bereich der ebenflächigen Abschnitte und/oder der ebenflächigen Streben (7) mit dem Ballon (3) verbunden ist.

2. Ballonkathetervorrichtung (1) gemäß Anspruch 1,
**dadurch gekennzeichnet,**
**dass** die Funktionshülse (5) aus einem gering dehnfähigem Polymer ausgebildet ist, dessen Dehnfähigkeit geringer als die Dehnfähigkeit der Ballonmembran des Ballons (3) ist.

3. Ballonkathetervorrichtung (1) gemäß Anspruch 1 oder 2,
**dadurch gekennzeichnet,**
**dass** die Hülse einen Durchmesser aufweist, der in etwa einem Durchmesser des expandierten Ballons entspricht.

4. Ballonkathetervorrichtung (1) gemäß einem der Ansprüche 1 bis 3,
**dadurch gekennzeichnet,**
**dass** die Verbindung der Hülse (5) mit der Ballonmembran z.B. durch punktuelles Polymer-Schweißen oder Kleben, wobei die Verbindungspunkte bevorzugt auf inneren Faltungslinien liegen, oder durch flächige Adhärenz eines wirkstoffhaltigen, adhäsiven Gels zwischen Ballonmembran und Funktionshülse erfolgt, wobei die Verbindung zwischen Ballon und Hülse vorzugsweise durch die Adhärenz einer wirkstoffhaltigen adhäsiven Schicht, die sich zwischen dem Ballon und der Funktionshülse angeordnet ist erfolgt, wobei zusätzlich und oder alternativ eine punktuelle und/oder linienförmige Verbindung durch Laserschweißen, oder eine flächige Verbindung durch Kleben, insbesondere eine Verbindung über alle ebenmäßigen Flächen oder Streben vorgesehen ist.

5. Ballonkathetervorrichtung (1) gemäß einem der Ansprüche 1 bis 4,
**dadurch gekennzeichnet,**
**dass** die Durchgangsöffnungen als insbesondere lasergeschnittene Schlitze ausgebildet sind, wobei diese Schlitze eine vorzugsweise eine axial orientierte sinusförmige Konfiguration haben,

6. Ballonkathetervorrichtung (1) gemäß einem der Ansprüche 1 bis 4,
**dadurch gekennzeichnet,**
**dass** die Durchgangsöffnungen als insbesondere lasergeschnittene Schlitze ausgebildet sind, wobei in der Funktionshülse (5) in einer Axialrichtung der Funktionshülse alternierend Abschnitte mit Axialschlitzen, die als Axialabschnitte bezeichnet werden, und Abschnitte mit Tangentialschlitzen, die als Tangentialabschnitte bezeichnet werden, ausgebildet sind, und wobei die Axialabschnitte und die Tangentialabschnitte beabstandet zueinander angeordnet sind.

7. Ballonkathetervorrichtung (1) gemäß Anspruch 6,
**dadurch gekennzeichnet,**
**dass** es durch das Vorsehen von alternierend angeordneten Axialabschnitten mit entsprechenden Axialschlitzen und Tangentialabschnitten mit entsprechenden Tangentialschlitzen zu einer konstanten bzw. im Wesentlichen gleichförmigen Aufweitung eines Durchmessers der Hülse und des Ballons in Radialrichtung kommt.

8. Ballonkathetervorrichtung (1) gemäß Anspruch 6 oder 7,
**dadurch gekennzeichnet,**
**dass** die Tangentialabschnitte Entkopplungsabschnitte zwischen den Axialabschnitten ausbilden, um einen in etwa konstanten Durchmesser in radialer Richtung über eine im Wesentlichen gesamte Länge der Vorrichtung in Axialrichtung bereitzustellen, sodass die Vorrichtung in Axialrichtung im Wesentlichen zylindrisch aufweitbar ist.

9. Ballonkathetervorrichtung (1) gemäß einem der Ansprüche 6 bis 8,
**dadurch gekennzeichnet,**
**dass** die alternierend angeordneten Axialabschnitte und Tangentialabschnitte in einer Axialrichtung derart ausgebildet und beabstandet zueinander angeordnet, dass sie einander nicht überlappen, und/oder dass die Axialschlitze der Axialabschnitte und die Tangentialschlitze der Tangentialabschnitte derart ausgebildet und angeordnet sein, dass sie einander in Tangentialrichtung nicht überlappen und/oder
**dass** durch die durch die Tangentialabschnitte ausgebildeten Entkopplungsabschnitte eine Einschnürung des Ballons in diesen Bereichen erfolgt, sodass ein im Wesentlichen konstanter Durchmesser in den Axialabschnitten bereitgestellt wird, sodass es bei einer Entfaltung zu einer im Wesentlichen gleichmäßigen Verteilung der Kräfte in radialer Richtung nach außen kommt.

10. Ballonkathetervorrichtung (1) gemäß einem der Ansprüche 1 bis 9,
**dadurch gekennzeichnet,**
**dass** zwischen der Funktionshülse (5) und dem Ballon (3) eine medizinische Wirkstoffschicht (4) mit einem medizinischen Wirkstoff angeordnet ist, wobei die medizinische Wirkstoffschicht (4) als eine Beschichtung der Ballonmembran aus einem, insbesondere biodegradierbaren, Polymer-Gel und einem medizinischen Wirkstoff ausgebildet ist, sodass der medizinische Wirkstoff in einer Polymer-Gel-Formulierung enthalten ist, und/oder
einem auf der Ballonmembran aufgesprühten, insbesondere biodegradierbaren, Polymer-Vlies mit dem medizinischen Wirkstoff, und/oder
als rohrförmige Zwischenhülse mit einer Vliesstruktur aus, insbesondere biodegradierbaren, Polymer und dem medizinischen Wirkstoff ausgebildet ist, sodass
die Funktionshülse (5) zum Schutz der unter der Funktionshülse (5) angeordneten Wirkstoffschicht ausgebildet ist und im Bereich der Durchgangsöffnungen (6) einen zweiten Funktionsbereich zur Wirkstoffabgabe ausbildet.

11. Ballonkathetervorrichtung (1) gemäß Anspruch 10,
**dadurch gekennzeichnet,**
**dass** der medizinische Wirkstoff vorzugsweise in ein Polymer der medizinischen Wirkstoffschicht (4) eingelagert ist, und wobei
der medizinische Wirkstoff vorzugsweise ein Antiproliferativum, bspw. Sirolimus, oder andere Limus-Derivate oder Paclitaxel (PTX) oder ein langzeitstabiles Depot-Gestagen, bspw. Etonogestrel, Levonorgestrel oder ein Antiprogesteron, bspw. Mifepriston oder ein Spermizid bspw. Nonoxinol 9 oder ein Zytostatikum, bspw. Mitomycin, Capecitabin oder Methotrexat (MTX) ist.

12. Ballonkathetervorrichtung (1) gemäß einem der Ansprüche 1 bis 11,
**dadurch gekennzeichnet,**
**dass** die gesamte Funktionshülse (5) und somit die die Funktionshülse (5) ausbildenden ebenflächigen Streben (7) Poren (16) aufweisen, sodass die Funktionshülse (5) über Ihre gesamte Mantelwandung den zweiten Funktionsbereich zur Wirkstoffabgabe ausbildet.

13. Ballonkathetervorrichtung (1) gemäß einem der Ansprüche 1 bis 126,
**dadurch gekennzeichnet,**
**dass** die Hülse zusammen mit der Ballonmembran entfaltbar und zusammenfaltbar ist.

14. Verfahren zur Herstellung einer Funktionshülse (5) für eine Ballonkathetervorrichtung (1) gemäß einem der Ansprüche 1 bis 13, umfassend die zeitlich nacheinander folgenden Schritte:
Blasformen eines rohrförmigen Polymer-Rohlings in einer temperierten metallischen Form auf den Durchmesser der inneren Mantelwandung,
Entformung und Ablängung im Bereich von Hülsenschultern,
Aufziehen der Hülse auf einen entfaltbaren und/oder komprimierbaren Haltekern, Schneiden, insbesondere Laserschneiden, von Durchgangsöffnungen (6) in die Hülse, um die Funktionshülse (5) auszubilden.

15. Verfahren gemäß Anspruch 14 zur Herstellung einer Ballonkathetervorrichtung (1) gemäß einem der Ansprüche 1 bis 13, umfassend die folgenden Schritte:
Aufbringen einer medizinischen Wirkstoffschicht (4) auf eine äußere Mantelwandung eines vorzugsweise vorgefalteten Ballons (3) einer Ballonkathetervorrichtung (1) im aufgedehnten Zustand des Bal-Ions (3),
Aufbringen einer vorgefalteten Funktionshülse (5) auf/über die getrocknete Wirkstoffschicht bei deflatiertem Ballon (3), derart dass die Falten von Ballon (3) und Hülse (5) ineinander liegen,
Verbinden der Funktionshülse (5) und des Ballons (3),
Gleichmäßiges Falten und Wickeln des Ballons (3) gemeinsam mit der Funktionshülse (5) um eine Längsachse der Ballonkathetervorrichtung (1) für die Lagerung und bei Anwendung für den Transport zum Wirkort.

## Claims

1. A balloon catheter device (1) for the atraumatic dilation of hollow organs, comprising a balloon catheter (2) with a balloon (3),
a tubular functional sleeve (5) applied to the balloon (3) for the directional, segmented, cushion-like unfolding of the balloon (3),
wherein the functional sleeve (5) is folded in an initial state together with the balloon (3) about a longitudinal axis of the sleeve, wherein the folding of the functional sleeve (5) is directed as a pleating about a sleeve longitudinal axis and is unfoldable in a final state for abutting against an inner wall of a hollow organ, and wherein the tubular functional sleeve (5) has planar sections and/or planar struts (7) which delimit through-openings (6), **characterized in that** the functional sleeve (5) is configured to sectionally confine an expansion of the balloon (3), wherein the functional sleeve (5) is connected to the balloon (3) in the region of the planar sections and/or the planar struts (7).

2. The balloon catheter device (1) according to claim 1,
**characterized in that**
the functional sleeve (5) is formed from a polymer with low stretchability, the stretchablility of which is lower than the stretchability of the balloon membrane of the balloon (3).

3. The balloon catheter device (1) according to claim 1 or 2,
**characterized in that**
the sleeve (5) has a diameter that corresponds approximately to the diameter of the expanded balloon.

4. A balloon catheter device (1) according to any one of claims 1 through 3,
**characterized in that**
the connection of the sleeve (5) to the balloon membrane is made, for example, by spot polymer welding or gluing, wherein the connection points are preferably located on inner folding lines, or by surface adhesion of an active-ingredient-containing adhesive gel between the balloon membrane and the functional sheath, wherein the connection between the balloon and the sleeve is preferably achieved by the adhesion of an active-ingredient-containing adhesive layer arranged between the balloon and the functional sleeve, wherein additionally and/or alternatively a spot and/or linear connection by laser welding, or a planar connection by adhesive bonding, in particular a connection over all planar surfaces or struts, is provided.

5. A balloon catheter device (1) according to any one of claims 1 through 4,
**characterized in that**
the through-openings (6) are formed as slits, in particular laser-cut slits, wherein these slits have a preferably axially oriented sinusoidal configuration.

6. A balloon catheter device (1) according to any one of claims 1 through 4,
**characterized in that**
the through-openings (6) are formed as, in particular, laser-cut slits, wherein in the functional sleeve (5), sections with axial slits, referred to as axial sections, and sections with tangential slits, referred to as tangential sections, are formed alternately in the axial direction of the functional sleeve, and wherein the axial sections and the tangential sections are arranged spaced from one another.

7. The balloon catheter device (1) according to claim 6,
**characterized in that**
alternately arranged axial sections with corresponding axial slits and tangential sections with corresponding tangential slits are provided which results in a constant or essentially uniform widening of the diameter of the sleeve and the balloon in a radial direction.

8. The balloon catheter device (1) according to claim 6 or 7,
**characterized in that**
the tangential sections form decoupling sections between the axial sections in order to provide an approximately constant diameter in the radial direction over a substantially entire length of the device in the axial direction, so that the device can be expanded substantially cylindrically in the axial direction.

9. The balloon catheter device (1) according to any one of claims 6 through 8,
**characterized in that**
the alternately arranged axial sections and tangential sections are formed and arranged at a distance from one another in an axial direction in such that they do not overlap one another, and/or that the axial slits of the axial sections and the tangential slits of the tangential sections are formed and arranged in such that they do not overlap one another in the tangential direction and/or
the decoupling sections formed by the tangential sections cause a constriction of the balloon diameter in these areas so that a substantially constant diameter is provided in the axial sections, resulting in a substantially uniform distribution of forces in the radial direction towards the outside during unfolding.

10. The balloon catheter device (1) according to any one of claims 1 through 9,
**characterized in that**
a medical active substance layer (4) containing a medical active substance is arranged between the functional sleeve (5) and the balloon (3), wherein the medical active substance layer (4) is formed as a coating of the balloon membrane formed from a polymer gel, in particular a biodegradable polymer gel, and a medical active substance, so that the medical active substance is contained in a polymer gel formulation, and/or
a polymer non-woven applied onto the balloon membrane, in particular a biodegradable polymer non-woven with the medical active ingredient, and/or
as a tubular intermediate sleeve with a non-woven structure made of, in particular biodegradable polymer and the medical active ingredient, so that
the functional sleeve (5) is designed to provide protection of the active ingredient layer arranged under the functional sleeve (5) and forms a second functional area for releasing the active ingredient in the region of the through-openings (6).

11. The balloon catheter device (1) according to claim 10,
**characterized in that**
the medical active ingredient is preferably incorporated in a polymer of the medical active ingredient layer (4), and wherein
the active medical ingredient is preferably an antiproliferative, e.g. sirolimus, or other limus derivatives, or paclitaxel (PTX), or a long-term stable depot progestogen, e.g. etonogestrel, levonorgestrel, or an antiprogesterone, e.g. mifepristone, or a spermicide, e.g. nonoxinol 9, or a cytostatic agent, mitomycin, capecitabine or methotrexate (MTX).

12. The balloon catheter device (1) according to any one of claims 1 through 11,
**characterized in that**
the entire functional sleeve (5), and thus the planar struts (7) forming the functional sleeve, have pores (16), so that the functional sleeve (5) forms the second functional area for active substance release over its entire circumferential wall.

13. The balloon catheter device (1) according to any one of claims 1 through 12,
**characterized in that**
the sleeve is unfoldable and foldable together with the balloon membrane.

14. A method for manufacturing a functional sleeve (5) for a balloon catheter device (1) according to any one of claims 1 through 13, comprising the following steps in chronological order:
- blow molding of a tubular polymer blank in a temperature-controlled metal mold to the diameter of the inner wall,
- demolding and cutting to length in the area of the sleeve shoulders,
- mounting the sleeve onto an expandable and/or compressible holding mandrel,
- cutting, in particular laser cutting, of through-openings (6) in the sleeve to form the functional sleeve (5).

15. A method according to claim 14 for manufacturing a balloon catheter device (1) according to any one of claims 1 through 13, comprising the following steps:
- applying a medical active substance layer (4) to an outer circumferential wall of a preferably pre-folded balloon (3) of a balloon catheter device (1) in the expanded state of the balloon (3),
- applying a pre-folded functional sleeve (5) onto/over the dried active substance layer (4) with the balloon deflated, so that the folds of the balloon (3) and sleeve (5) lie inside each other,
- connecting the functional sleeve (5) and the balloon (3),
- uniform folding and wrapping of the balloon (3) together with the functional sleeve (5) around a longitudinal axis of the balloon catheter device (1) for storage and, when applied, for transportation to the site of action.

## Revendications

1. Dispositif de cathéter à ballonnet (1) destiné à la dilatation atraumatique d'organes creux, comprenant un cathéter à ballonnet (2) muni d'un ballonnet (3),
une gaine fonctionnelle tubulaire (5) appliquée sur le ballonnet (3) pour permettre un déploiement dirigé et segmenté du ballonnet (3) à la manière d'un accordéon,
dans lequel
la gaine fonctionnelle (5) est, dans un état initial, pliée avec le ballon (3) autour d'un axe longitudinal de la gaine, le pliage de la gaine fonctionnelle (5) est orienté sous forme de plissage autour d'un axe longitudinal de la gaine et peut être déployé dans un état final pour venir en appui contre une paroi interne d'un organe creux, et la gaine fonctionnelle tubulaire (5) présentant des sections planes et/ou des entretoises planes (7) qui délimitent des ouvertures de passage (6), **caractérisé en ce que** la gaine fonctionnelle (5) est conçue pour limiter par sections une dilatation du ballon (3), la gaine fonctionnelle (5) étant reliée au ballon (3) au niveau des sections planes et/ou des entretoises planes (7).

2. Dispositif de cathéter à ballonnet (1) selon la revendication 1,
**caractérisé en ce**
**que** la gaine fonctionnelle (5) est réalisée en un polymère peu extensible, dont l'extensibilité est inférieure à celle de la membrane du ballonnet (3).

3. Dispositif de cathéter à ballonnet (1) selon la revendication 1 ou 2,
**caractérisé en ce**
**que** la gaine présente un diamètre qui correspond approximativement au diamètre du ballonnet expansé.

4. Dispositif de cathéter à ballonnet (1) selon l'une des revendications 1 à 3,
**caractérisé en ce**
**que** la liaison entre la gaine (5) et la membrane du ballon est réalisée, par exemple, par soudage ponctuel de polymères ou par collage, les points de liaison se situant de préférence sur des lignes de pliage internes, ou par adhérence sur toute la surface d'un gel adhésif contenant un principe actif entre la membrane du ballon et la gaine fonctionnelle, la liaison entre le ballon et la gaine s'effectuant de préférence par l'adhérence d'une couche adhésive contenant un principe actif, disposée entre le ballon et la gaine fonctionnelle, une liaison ponctuelle et/ou linéaire par soudage au laser, ou une liaison sur toute la surface par collage, en particulier une liaison sur toutes les surfaces planes ou les entretoises, étant en outre et/ou en alternative prévue.

5. Dispositif de cathéter à ballonnet (1) selon l'une des revendications 1 à 4,
**caractérisé en ce**
**que** les ouvertures de passage sont réalisées sous forme de fentes, en particulier découpées au laser, ces fentes présentant de préférence une configuration sinusoïdale orientée axialement.

6. Dispositif à cathéter à ballonnet (1) selon l'une des revendications 1 à 4,
**caractérisé en ce**
**que** les ouvertures de passage sont réalisées sous forme de fentes, notamment découpées au laser, des sections comportant des fentes axiales, appelées sections axiales, et des sections comportant des fentes tangentielles, désignées comme sections tangentielles, et les sections axiales et les sections tangentielles étant disposées à distance les unes des autres.

7. Dispositif de cathéter à ballonnet (1) selon la revendication 6,
**caractérisé en ce**
**que** la disposition en alternance de sections axiales comportant des fentes axiales correspondantes et de sections tangentielles comportant des fentes tangentielles correspondantes permet d'obtenir un élargissement constant ou sensiblement uniforme du diamètre de la gaine et du ballonnet dans la direction radiale.

8. Dispositif de cathéter à ballonnet (1) selon la revendication 6 ou 7,
**caractérisé en ce**
**que** les sections tangentielles forment des sections de découplage entre les sections axiales afin de fournir un diamètre sensiblement constant dans la direction radiale sur pratiquement toute la longueur du dispositif dans la direction axiale, de sorte que le dispositif est sensiblement expansible de manière cylindrique dans la direction axiale.

9. Dispositif à cathéter à ballonnet (1) selon l'une des revendications 6 à 8,
**caractérisé en ce**
**que** les sections axiales et les sections tangentielles disposées en alternance sont conçues et espacées les unes des autres dans une direction axiale de telle sorte qu'elles ne se chevauchent pas, et/ou en ce que les fentes axiales des sections axiales et les fentes tangentielles des sections tangentielles sont formées et disposées de telle sorte qu'elles ne se chevauchent pas dans la direction tangentielle et/ou
**que** les sections de découplage formées par les sections tangentielles provoquent un rétrécissement du ballon dans ces zones, de sorte qu'un diamètre essentiellement constant est obtenu dans les sections axiales, ce qui permet, lors du déploiement, une répartition essentiellement uniforme des forces dans la direction radiale vers l'extérieur.

10. Dispositif de cathéter à ballonnet (1) selon l'une des revendications 1 à 9,
**caractérisé en ce**
**qu'**une couche de principe actif médical (4) contenant un principe actif médical est disposée entre la gaine fonctionnelle (5) et le ballonnet (3), la couche de principe actif médical (4) étant constituée d' un revêtement de la membrane du ballonnet constitué d'un gel polymère, en particulier biodégradable, et d'un principe actif médical, de sorte que le principe actif médical est contenu dans une formulation de gel polymère, et/ou
d'un non-tissé polymère, en particulier biodégradable, imprégné du principe actif médical et pulvérisé sur la membrane du ballonnet, et/ou
est réalisé sous la forme d'une gaine intermédiaire tubulaire comportant une structure en non-tissé constituée d'un polymère, notamment biodégradable, et du principe actif médical, de sorte que la gaine fonctionnelle (5) est conçue pour protéger la couche de principe actif disposée sous la gaine fonctionnelle (5) et forme, au niveau des ouvertures de passage (6), une deuxième zone fonctionnelle destinée à la libération du principe actif.

11. Dispositif à cathéter à ballonnet (1) selon la revendication 10,
**caractérisé en ce**
**que** le principe actif médical est de préférence incorporé dans un polymère de la couche de principe actif médical (4), et dans lequel
le principe actif médical est de préférence un agent antiprolifératif, par exemple le sirolimus, ou d'autres dérivés du limus, ou le paclitaxel (PTX), ou un progestatif à libération prolongée stable, par exemple l'étonogestrel, le lévonorgestrel, ou un antiprogestérone, par exemple la mifépristone, ou un spermicide, par exemple le nonoxinol 9, ou un cytostatique, par exemple la mitomycine, la capécitabine ou le méthotrexate (MTX).

12. Dispositif de cathéter à ballonnet (1) selon l'une des revendications 1 à 11,
**caractérisé en ce**
**que** l'ensemble de la gaine fonctionnelle (5) et, par conséquent, les entretoises à surface plane formant la gaine fonctionnelle (5) (7) présentent des pores (16), de sorte que la gaine fonctionnelle (5) forme, sur toute la surface de sa paroi, la deuxième zone fonctionnelle destinée à la libération du principe actif.

13. Dispositif de cathéter à ballonnet (1) selon l'une des revendications 1 à 12,
**caractérisé en ce**
**que** la gaine peut être déployée et repliée conjointement avec la membrane du ballonnet..

14. Procédé de fabrication d'une gaine fonctionnelle (5) pour un dispositif de cathéter à ballonnet (1) selon l'une des revendications 1 à 13, comprenant les étapes successives suivantes:
- moulage par soufflage d'une ébauche tubulaire en polymère dans un moule métallique tempéré au diamètre de la paroi interne de l'enveloppe,
- démoulage et tronçonnage au niveau des épaulements de la gaine,
- enfilage de la gaine sur un noyau de maintien dépliable et/ou compressible,
- découpe, en particulier par laser, d'ouvertures de passage (6) dans la gaine afin de former la gaine fonctionnelle (5).

15. Procédé selon la revendication 14 pour la fabrication d'un dispositif de cathéter à ballonnet (1) selon l'une des revendications 1 à 13, comprenant les étapes suivantes:
- application d'une couche de principe actif médical (4) sur une paroi externe d'enveloppe d'un ballonnet (3) - de préférence pré-plié - d'un dispositif de cathéter à ballonnet (1) lorsque le ballonnet est à l'état gonflé (3),
- l'application d'une gaine fonctionnelle pré-pliée (5) sur/au-dessus de la couche de principe actif séchée, le ballon (3) étant dégonflé, de telle sorte que les plis du ballon (3) et de la gaine (5) s'emboîtent les uns dans les autres,
- l'assemblage de la gaine fonctionnelle (5) et du ballon (3),
- pliage et enroulement réguliers du ballon (3) avec la gaine fonctionnelle (5) autour d'un axe longitudinal du dispositif de cathéter à ballon (1) pour le stockage et, lors de l'utilisation, pour le transport vers le site d'action.
